Europäisches Patentamt

European Patent Office

Office européen des brevets

(19)

(11) **EP 0 897 540 B1**

(12) **FASCICULE DE BREVET EUROPEEN**

(45) Date de publication et mention
de la délivrance du brevet:
**04.07.2001 Bulletin 2001/27**

(51) Int Cl.⁷: **G01N 33/53**, C12Q 1/34,
G01N 33/52

(21) Numéro de dépôt: **97917955.3**

(22) Date de dépôt: **30.04.1997**

(86) Numéro de dépôt international:
**PCT/BE97/00052**

(87) Numéro de publication internationale:
**WO 97/41435 (06.11.1997 Gazette 1997/47)**

(54) **PROCEDE DE DETECTION ET/OU DE QUANTIFICATION D'UN HAPTENE DANS UNE PHASE HOMOGENE ET DISPOSITIF POUR SA MISE EN OEUVRE**

VERFAHREN ZUM NACHWEIS UND/ODER QUANTIFIKATION EINES HAPTENS IN EINER HOMOGENEN PHASE UND VORRICHTUNG DAFÜR

METHOD FOR DETECTING AND/OR QUANTIFYING A HAPTEN IN A HOMOGENEOUS PHASE AND DEVICE FOR IMPLEMENTATION THEREOF

(84) Etats contractants désignés:
**AT BE CH DE ES FR GB IT LI NL SE**

(30) Priorité: **30.04.1996 BE 9600384**

(43) Date de publication de la demande:
**24.02.1999 Bulletin 1999/08**

(73) Titulaire: **LA REGION WALLONNE**
**1040 Bruxelles (BE)**

(72) Inventeurs:
• **KOHL, Michel**
  **B-4000 Liège (BE)**
• **RENOTTE, Roger**
  **B-4360 Oreye (BE)**
• **GHITTI, Gianangelo**
  **B-4000 Liège (BE)**
• **SARLET, Guy**
  **B-4100 Seraing (BE)**
• **LEJEUNE, Robert**
  **B-4802 Heusy (BE)**

(74) Mandataire: **Van Malderen, Michel**
  **Office van Malderen**
  **85/043 Boulevard de la Sauvenière**
  **4000 Liège (BE)**

(56) Documents cités:
  **EP-A- 0 117 648        EP-A- 0 532 187**
  **FR-A- 2 339 172        US-A- 4 764 462**

• **JOURNAL OF CHROMATOGRAPHY, vol. 489, no. 1, 1989, pages 235-243, XP000616710 G. DEGAND ET AL.: "Enzyme immunoassay screening procedure for the synthetic anabolic estrogens diethylstilbestrol, nortestosterone, methyltestosterone and trenbolone in bovine urine."**

## Description

### Objet de l'invention

La présente invention concerne un procédé de détection et/ou de quantification d'un haptène dans une phase homogène, ainsi que le dispositif de détection et/ou de quantification, en particulier la trousse permettant le diagnostic et/ou le dosage d'un haptène dans une phase homogène.

### Arrière-plan technologique à la base de l'invention

Ces dernières années, les importants progrès dans le domaine des biotechnologies ont permis de développer des immuno-essais permettant le dosage en phase homogène d'haptènes, c'est-à-dire des molécules de petite taille d'origine naturelle ou obtenues par voie synthétique, et utilisées notamment pour traiter des animaux ou des humains.
De telles molécules peuvent être par exemple des composants actifs de médicaments, des hormones, des anabolisants, ... .
Pour faciliter l'application des tests de diagnostic et/ou de dosage de ces haptènes, on a cherché à développer des immuno-essais pouvant être utilisés en phase homogène.
C'est ainsi que des immunoassay enzymatiques ont été proposés. Il s'agit des "substrate labelled fluorescent immunoassay", "apoenzyme reactivation immunoassay system", "enzyme multiplied immunoassay test" et "enzyme channeling immunoassay" décrits par Voiler et Bidwell (Voller A., Bidwell D. E., in Alternative Immunoassays, Collins WP Ed. John Wiley, Chichester, pp. 78-79 (1986)) ainsi que le cloned enzyme donnor immunoassay (Henderson et al., Cli. Chem., 32, 9, pp. 1637-1641 (1986)).
La demande de brevet EP-0117648 décrit un immuno-dosage de stéroïdes en phase basé sur l'inhibition de la précipitation (clotting) du lait, ce dosage donnant une indication de la fertilité des animaux domestiques producteurs de lait.
Cependant, à ce jour, on n'a pas réussi à développer des immuno-dosages travaillant en phase homogène qui soient suffisamment sensibles, spécifiques et reproductibles pour permettre leur utilisation courante dans le dosage et/ou la détection d'un grand nombre d'haptènes en biologie clinique et dans différents milieux (sang, urine, etc.).
Les systèmes utilisés jusqu'à présent à base d'iode présentent l'inconvénient que pratiquement tous les iodures sont oxydables à l'air en générant de l'iode qui est perdu par sublimation. Ceci constitue donc une première source d'instabilité. D'autre part, l'empois d'amidon est facilement dégradé par des bactéries ou des champignons, ce qui constitue une autre source d'instabilité.
La demande de brevet FR-2339172 décrit un système réactif pour déterminer si de l'acide urique ou une autre matière oxydable par l'iode se trouve dans un liquide dans une proportion supérieure à une quantité prédéterminée en milieu alcalin, ledit réactif comprenant un générateur d'iode activable par de l'eau, pouvant libérer in situ une quantité appropriée d'iode libre avec un indicateur pour déceler la présence de l'iode. Le générateur d'iode et l'indicateur sont appliqués sur une bande d'essai. Par conséquent, un tel dispositif fonctionne uniquement à l'état sec et reste donc inféodé à une analyse sur système solide.

### Buts de l'invention

La présente invention vise à obtenir un procédé et un dispositif de diagnostic et/ou de dosage d'un haptène dans tout type de phase homogène (sang, sérum, urine, lait, etc.) qui soient suffisamment sensibles, spécifiques et reproductibles.
En particulier, on cherche à obtenir un procédé et un dispositif qui permettent notamment de détecter des haptènes présents dans un liquide physiologique humain ou animal, à des concentrations de l'ordre de 100 pM ou supérieures, voire à des concentrations de l'ordre de 10 pM ou supérieures.
Un but particulier de la présente invention vise à optimiser la sensibilité desdits procédé et dispositif en réduisant le "bruit de fond" observé dans les dispositifs et procédés de l'état de la technique.
Un dernier but de la présente invention vise à obtenir un dispositif qui serait susceptible d'être conservé plus longtemps sans contamination ni dégradation.

### Principaux éléments caractéristiques de l'invention

La présente invention concerne un procédé de détection et/ou de quantification d'un haptène dans une phase homogène, dans lequel :

- on additionne une quantité connue d'un complexe inhibiteur - haptène à la solution contenant l'haptène à détecter et/ou à quantifier;

- on additionne à la solution une quantité d'anticorps correspondant à la quantité du complexe inhibiteur-haptène;
- on additionne à la solution une β-lactamase de type C possédant un site actif pour deux substrats entrant en compétition sur ledit site actif, le premier substrat étant un substrat reporter transformable en un produit détectable et/ou quantifiable, de préférence par mesure de rayonnement UV - visible, le second substrat étant le complexe inhibiteur - haptène agissant sur la vitesse d'hydrolyse du substrat reporter;
- on détecte et/ou quantifie la concentration du produit issu de la transformation du substrat reporter, la constante Km du substrat reporter étant au moins 100 fois plus, de préférence 10000 fois plus, élevée que la constante Km du complexe inhibiteur - haptène, et la constante $k_{cat}$ du substrat reporter étant au moins 10 fois plus, de préférence 10000 fois plus, élevée que la constante $k_{cat}$ du complexe inhibiteur - haptène.

Préalablement aux opérations précitées, on peut éventuellement ajouter à la solution contenant l'haptène à doser des substances en vue de lever les interférences éventuelles telles que des agents protecteurs de l'enzyme, des agents protecteurs du substrat reporter, des agents protecteurs du complexe haptène - inhibiteur ou des agents décontaminants, ... .

L'addition à la solution d'une quantité d'anticorps prémentionnée peut être combinée avec une opération d'addition à la solution d'un substrat reporter. Certaines ou la totalité des opérations précitées peuvent être combinées, c'est-à-dire effectuées simultanément, et/ou l'ordre des opérations indiqué peut être modifié.

C'est donc uniquement pour la clarté de la description que les différentes opérations préalables à l'addition d'une enzyme à la solution sont présentées successivement comme des étapes séparées.

Par conséquent, en l'absence d'haptène libre, toutes les molécules complexe inhibiteur - haptène auront fixé un anticorps et seront par conséquent inactives. L'activité enzymatique sera par conséquent maximale. Par contre, plus la quantité d'haptène à doser sera élevée, plus la quantité de complexe inhibiteur - haptène-anticorps sera faible, ce qui implique une disponibilité importante des molécules de complexe inhibiteur - haptène.

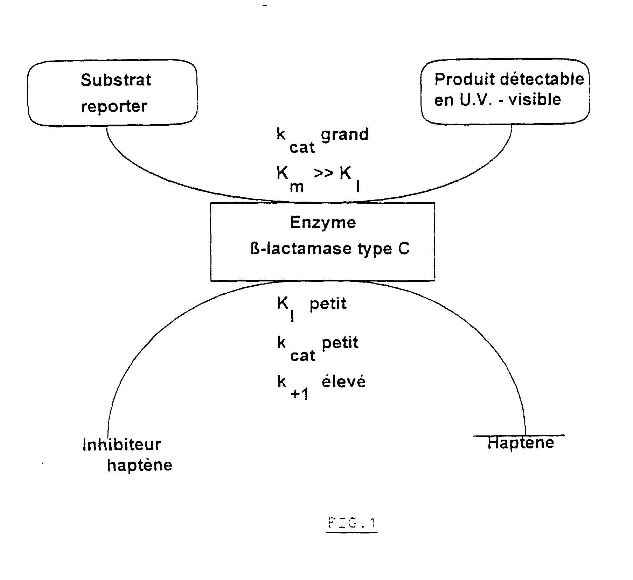

Il s'ensuivra une activité enzymatique faible, qui se traduira par une faible transformation du substrat reporter, qui sera détectée et/ou quantifiée par la faible absorption de rayonnement UV - visible (voir figure 1).

La présente invention concerne également le dispositif de détection et/ou de quantification d'un haptène dans une phase homogène, comprenant une enzyme possédant un site actif pour deux substrats entrant en compétition sur ledit site actif, ainsi que les deux substrats, le premier substrat étant un substrat reporter transformable en un produit détectable et/ou quantifiable, de préférence par mesure de rayonnement UV - visible, le second substrat étant un complexe inhibiteur - haptène modulant la vitesse d'hydrolyse du substrat reporter. Ledit dispositif comprend également des anticorps susceptibles de fixer ledit complexe inhibiteur - haptène.

La constante $k_{cat}$ du substrat reporter est élevée, supérieure à 0,1 s$^{-1}$, de préférence supérieure à 10 s$^{-1}$. La constante $K_i$ du complexe inhibiteur - haptène est faible, inférieure à 5000 µM, de préférence inférieure à 100 µM. La constante $k_{cat}$ du complexe inhibiteur-haptène est faible, inférieure à 0,1 s$^{-1}$.

Dans le procédé et le dispositif selon l'invention, l'enzyme est une β-lactamase, de préférence de type C. Avantageusement, la β-lactamase est choisie parmi le groupe constitué par les β-lactamases issues de *Enterobacter cloacae* Q908R et P99 et les β-lactamases issues de *Citrobacter freundii* et de *Escherichia coli.*

Selon l'invention, l'inhibiteur et le substrat reporter sont choisis parmi le groupe constitué par les pénicillines, les céphalosporines et les antibiotiques β-lactamiques.

Le substrat reporter est de préférence choisi parmi le groupe constitué par la céphaloridine, la nitrocéfine, la céphalothine, la céphalexine, la céphalosporine C, la céphacétrile et la céfazoline.

Selon l'invention, l'inhibiteur du complexe inhibiteur - haptène est de préférence choisi parmi le groupe constitué par la carbénicilline, l'oxacilline, la céfuroxine, le céfotaxime et la méthicilline.

De préférence, la mise en évidence de la transformation du substrat reporter est détectée par la mesure de la coloration propre des produits d'hydrolyse ou, lorsque ces produits ne sont pas colorés, par un système de révélation, en particulier par coloration à l'iode/amidon.

Il convient de noter que ce type de réaction peut convenir également à d'autres dosages, tels que le dosage de la céphalexine à l'iode, ainsi qu'il sera décrit dans un exemple d'exécution ci-après.

Dans le cas particulier de la détection et/ou la quantification (dosage) de la concentration du produit issu de la transformation du substrat reporter dont il a été question ci-dessus, cette coloration amidon/iode permet la détection et/ou le dosage d'un haptène à de faibles concentrations et dans une gamme de couleurs discriminante par rapport à la coloration générale de la phase homogène.

Dans le procédé et le dispositif selon l'invention, l'inhibiteur est de préférence choisi parmi le groupe constitué par la carbénicilline, l'oxacilline, la céfuroxine, le céfotaxime et la méthicilline, de même que toute autre substance à noyau β-lactamique ou apparenté au noyau β-lactamique, ou même toute substance ne possédant pas nécessairement un noyau β-lactamique ou apparenté au noyau β-lactamique mais présentant vis-à-vis du groupe enzyme précité des paramètres cinétiques mesurables ($K_m$, $K_i$ et $k_{cat}$) et assurant un ralentissement de la vitesse d'hydrolyse du substrat reporter.

Avantageusement, l'haptène à doser est un composant actif de médicament, une hormone, un anabolisant ou une drogue, de préférence choisie parmi le groupe constitué par la testostérone, l'oestridiol, la progestérone, l'aldostérone, le cortisol, la méthylamphétamine, la méthadone, le tétrahydrocannabinol, le $\Delta 4$ androsténédione, la morphine, le DHEA sulfate, la nandrolone, la théophylline, la cocaïne et/ou leurs dérivés d'hydrolyse.

## Description d'une forme d'exécution préférée de l'invention 1. Choix de l'enzyme

Les $\beta$-lactamases peuvent être regroupées en quatre familles : A, B, C et D. Celles des types A, C et D sont caractérisées par leur mode d'action impliquant la formation d'un complexe acyl-enzyme via un site actif comportant une sérine. Les enzymes de type C ont été avantageusement sélectionnées sur base :

- des paramètres cinétiques connus de la littérature,
- de leur schéma réactionnel en présence d'un inhibiteur,
- de leur disponibilité commerciale,
- de leur stabilité chimique et thermique.

Dans les réactions d'hydrolyse de pénicilline par des $\beta$-lactamases de type C, il n'existe pratiquement pas d'inhibiteurs francs. Les termes "bon et mauvais substrat" sont par conséquent utilisés pour caractériser les pénicillines dans leurs interactions avec les $\beta$-lactamases. Toutefois, par souci de simplification, les termes "substrat" et "inhibiteur" seront employés dans la suite du document.

Un bon substrat ou substrat reporter sera employé en conjugaison avec un mauvais substrat, c'est-à-dire avec le complexe haptène - inhibiteur. Les modèles d'inactivation par ce dernier postulent la formation d'un complexe enzyme - inhibiteur relativement stable par formation d'une acylenzyme.

Parmi les $\beta$-lactamases utilisables, on peut mentionner les $\beta$-lactamases d'*Enterobacter cloacae* Q908R et P99, de *Citrobacter freundii* ou de *Escherichia coli.*

### 2. Choix de l'inhibiteur

Le choix de l'inhibiteur repose sur des paramètres cinétiques et sur les possibilités de couplage avec l'haptène à doser (présence de groupements fonctionnels non indispensables à l'activité inhibitrice). Au niveau enzymatique, l'inhibiteur idéal doit présenter un $k_{cat}$ le plus faible possible (vitesse de dégradation très lente), tout en présentant un Km le plus petit possible (forte affinité pour l'enzyme). Dans le cas des réactions d'inhibition envisagées, la valeur du Km est proche de la constante $K_i$. Le Km peut donc être estimé à partir d'une évaluation du $K_i$. Ceci est obtenu à partir de mesures cinétiques réalisées en présence d'un substrat reporter de caractéristiques connues, et ce en présence d'une quantité variable d'inhibiteur. L'inhibiteur idéal doit également présenter une constante de vitesse $k_{+1}$ (correspondant à l'étape de fixation de l'inhibiteur sur l'enzyme) la plus élevée possible. Selon ce principe, cinq inhibiteurs ont pu être sélectionnés. Il s'agit de :

- la carbénicilline ($K_m$ = 0,5 à 20 nM; $k_{cat}$ = 0,002 à 0,004 s$^{-1}$),
- l'oxacilline ($K_m$ = 0,5 nM; $k_{cat}$ = 0,005 s$^{-1}$),
- la céfuroxine ($K_m$ = 20 nM; $k_{cat}$ = 0,04 à 0,15 s$^{-1}$),
- le céfotaxime ($K_m$ = 20 à 170 nM; $k_{cat}$ = 0,01 à 0,17 s$^{-1}$)
- la méthicilline ($K_m$ = 2 à 150 nM; $k_{cat}$ = 0,007 à 0,08 s$^{-1}$).

L'inhibiteur devra présenter au moins une fonction chimique libre et non essentielle à son activité inhibitrice permettant son couplage à l'haptène à doser.

L'inhibiteur utilisé doit de plus être susceptible d'être couplé à un stéroïde tel que la nandrolone, à un médicament tel que la théophylline ou à une drogue telle que la cocaïne.

### 3. Choix du substrat reporter

Les valeurs $K_m$ et $k_{cat}$ d'une série de substrats reporters mis en présence de diverses $\beta$-lactamases sont réunies dans les tableaux 1 et 2. Les données ont été soit déterminées expérimentalement, soit extraites de données disponibles dans la littérature.

EP 0 897 540 B1

| TABLEAU 1: PARAMETRES CINETIQUES DE DIFFERENTES PENICILLINES EN PRESENCE DE B-LACTAMASES | | | | | |
|---|---|---|---|---|---|
| Substrat | Enzyme[1] | $K_m (\mu M)$ | $k_{cat} (s^{-1})$ | $R_1$ | $R_2$ |
| Benzylpenicilline (pénicilline G) | P99/Q908R/Citro.F. | 0.5 | 20 | | Non |
| | E.Coli | 4.4 | 45 | | |
| Oxacilline | P99/Q908R/E.Coli | 0.0005 | 0.005 | | Non |
| Ticarcilline | **P99/Q908R/E.Coli** | **0.25** | 0.045 | | Non |
| Carbénicilline | **P99/Q908R** | **0.01** | **0.002** | | Non |
| | Citro.F. | 0.0005 | 0.004 | | |
| | E.Coli | 0.02 | 0.003 | | |
| Methicilline | P99/Q908R | 0.03 | 0.007 | | Non |
| | Citro.F. | 0.002 | 0.01 | | |
| | E.Coli | 0.15 | 0.08 | | |

[1] Citro.F. représente une β-lactamase de *Citrobacter freundii* et P99 et Q908R des β-lactamases provenant d' *Enterobacter cloacae*.

5

## TABLEAU 2 : PARAMETRES CINETIQUES DE DIFFERENTES CEPHALOSPORINES EN PRESENCE DE B-LACTAMASES

| Substrat | Enzyme | $K_m (\mu M)$ | $k_{cat} (s^{-1})$ | $R_1$ | $R_2$ |
|---|---|---|---|---|---|
| Céphaloridine | P99/Q908R | 70 | 650 | | |
| | Citro.F. | 35 | 700 | | |
| | E.Coli | 170 | 130 | | |
| Nitrocéphine | P99/Q908R | 25 | 780 | | |
| | Citro.F. | 12 | 300 | | |
| | E.Coli | 500 | 500 | | |
| Céphalothine | P99/Q908R | 15 | 200 | | $CH_3COOCH_2-$ |
| | E.Coli | 42 | 300 | | |
| Céphalexine | P99/Q908R | 10 | 100 | | $CH_3-$ |
| | Citro.F. | 12 | 170 | | |
| | E.Coli | 4 | 38 | | |
| Céphalosporine C (pénicill. N) | P99/Q908R | 400 | 1100 | | Non |
| Céphaglycine | P99/Q908R | 2 | 1 | | Non |
| Céphacetrile | P99/Q908R | 140 | 143 | $-CH_2-CN$ | Non |
| Céfuroxime | P99/Q908R | 0.02 | 0.04 | | $NH_2COOCH_2-$ |
| | E.Coli | 0.15 | 0.15 | | |
| Céfotaxime | P99/Q908R | 0.01 | 0.01 | | Non |
| | E.Coli | 1.7 | 0.17 | | |
| Céfazoline | P99/Q908R | 600-1500 ? | 3000 | | |
| | E.Coli | 400 | 150 | | |

On constate à la lecture des tableaux 1 et 2 que la céphaloridine, la nitrocéfine, la céphalotine, la céphalosporine C, le céphacétrile, la céphazoline (mais aussi l'apholexine) sont utilisables en tant que substrats reporters.

<u>4. Synthèse de carbénicillinate de nandrolone (conjugué 1)</u>

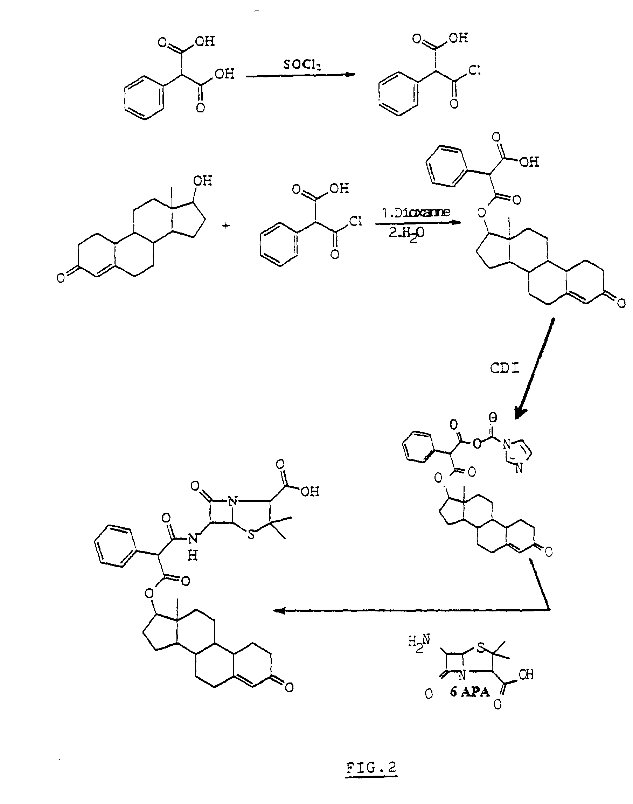

L'acide phénylmalonique est transformé en monochlorure d'acide par incubation à 35 °C dans un mélange éther - dioxanne sec avec 1,2 équivalents de chlorure de thionyle pendant 3 heures. Les solvants sont évaporés et le résidu (huile chaude), repris dans un mélange éther-dioxanne, est mélangé à une solution glacée de nandrolone dans le dioxanne. La mixture où le stéroïde est en défaut est incubée à température ambiante pendant 12 heures. Le produit est extrait par une solution aqueuse de bicarbonate de sodium, puis après acidification de la solution à pH 2, ré-extrait par du chloroforme. Le phénylmalonate de stéroïde est obtenu par évaporation du chloroforme (voir figure 2).
Le couplage du phénylmalonate de nandrolone au 6-APA est réalisé classiquement après activation au carbonyle diimidazole (CDI). La purification du conjugué est obtenue par extraction dans l'éther en milieu de pH 2 et 8,5 avant recristallisation finale d'un mélange toluène - pétroléine (voir tableau 9).

<u>5. Synthèse du carbénicillinate de cocaïne (conjugué 2)</u>

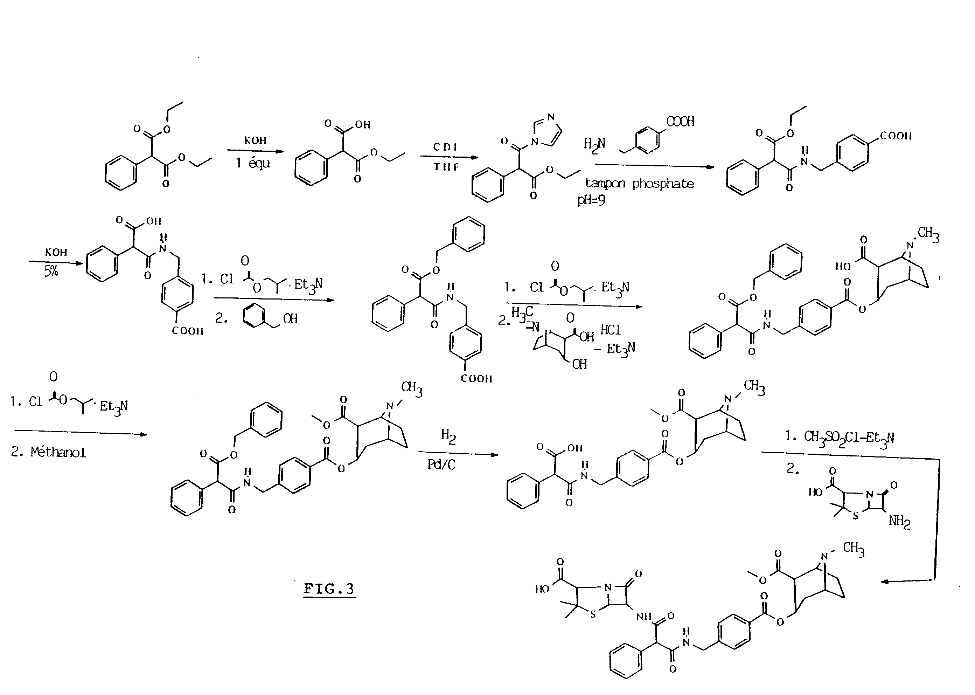

Le phénylmalonate de diéthyle est hydrolysé en monoester par action d'un équivalent de KOH en milieu hydro-alcoolique. Le monoester est ensuite conjugué à l'acide p-aminométhylbenzoïque par une procédure classique impliquant le carboxyldiimidazole comme agent activateur. L'ester éthylique résiduel est remplacé par un ester benzylique correspondant par hydrolyse au KOH 5% suivie d'une estérification par l'alcool benzylique en utilisant la méthode des anhydrides mixtes au chloroformate d'isobutyle en présence d'un équivalent de triéthylamine. La fonction carboxylique restante est activée classiquement par le chloroformate d'isobutyle puis couplée à l'ecgonine. L'acide carboxylique de la partie benzoylecgonine du conjugué est transformée par ester méthylique par la même technique appliquée en présence de méthanol. La fonction benzylique est enlevée par hydrogénolyse en présence de palladium sur charbon sous deux bars de pression d'hydrogène. L'acide généré est utilisé pour le couplage final au 6-APA en ayant recours à une activation préalable par le chlorure de méthanesulfonyle selon la méthode de Brown et al., Chem. Soc. Perkins Trans No. 1, p. 881 (1991) (voir figure 3). La purification de ce composé est obtenue par extraction dans l'éther en milieu de pH 2 et 8,5 avant recristallisation finale du mélange toluène - pétroléine (voir tableau 9).

<u>6. Synthèse de différents oxacillinates de nandrolone et d'autres stéroïdes</u>

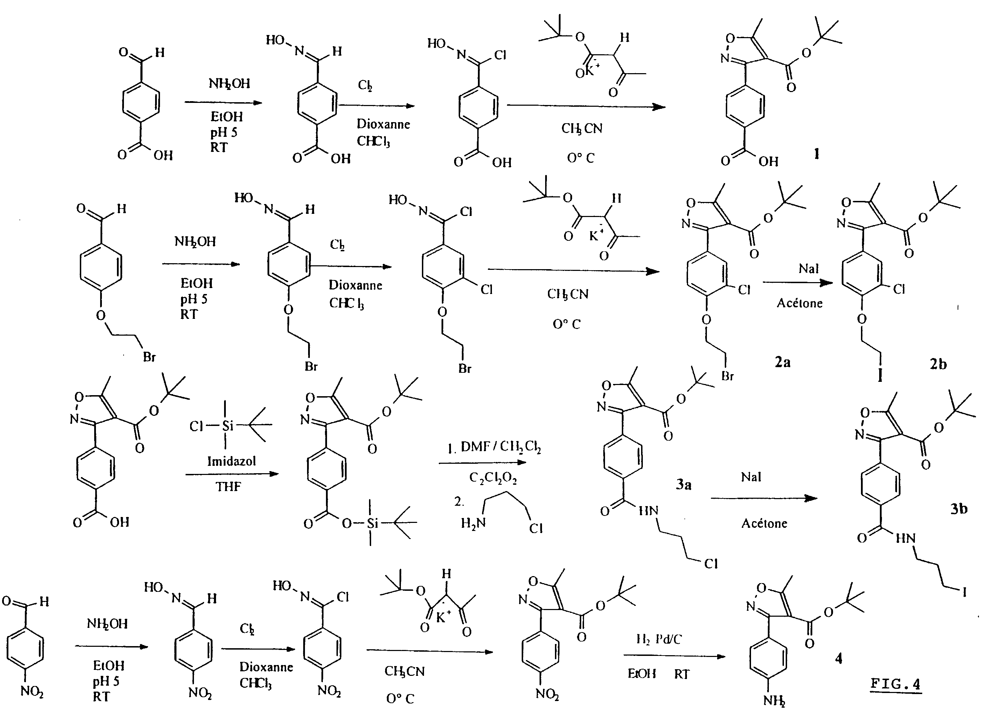

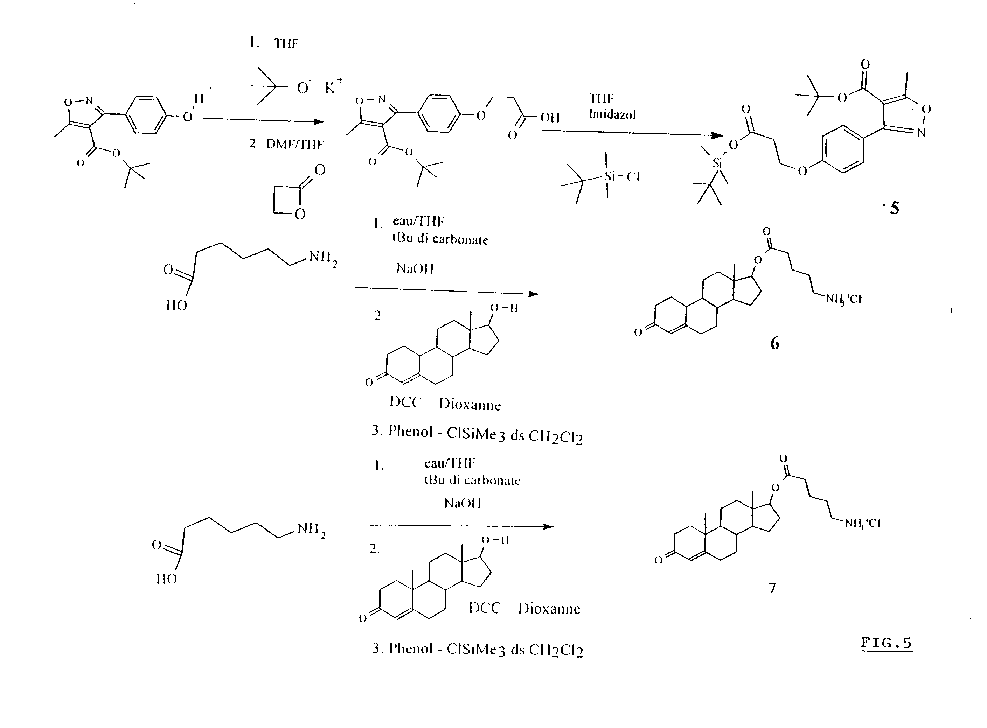

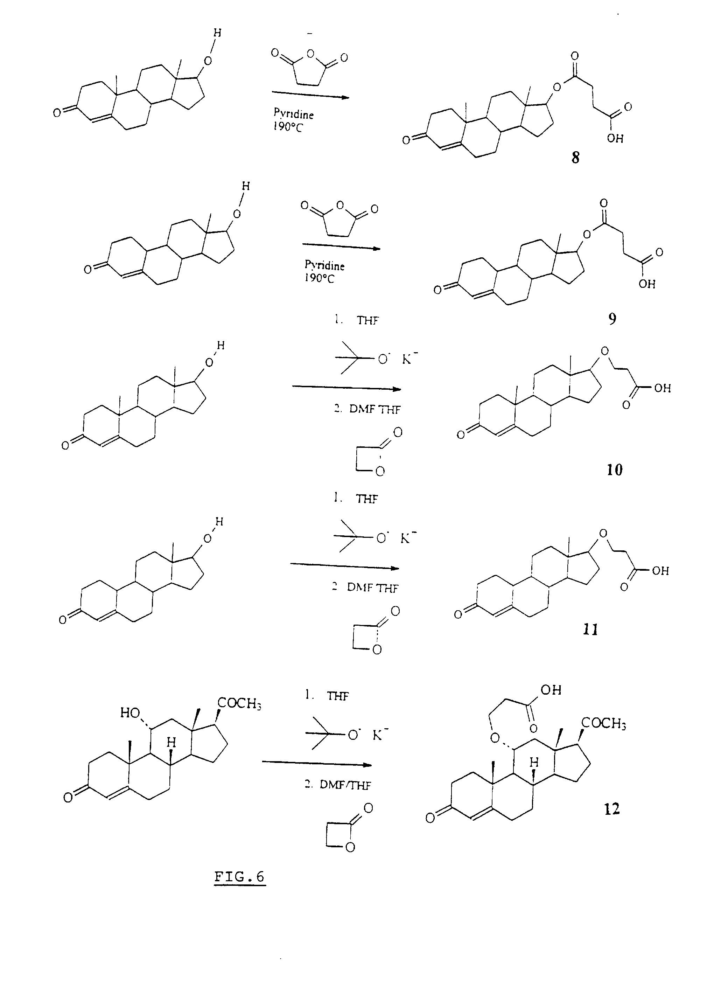

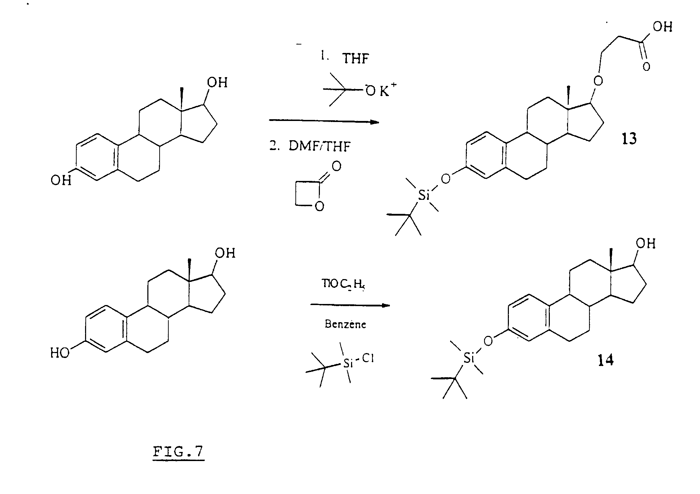

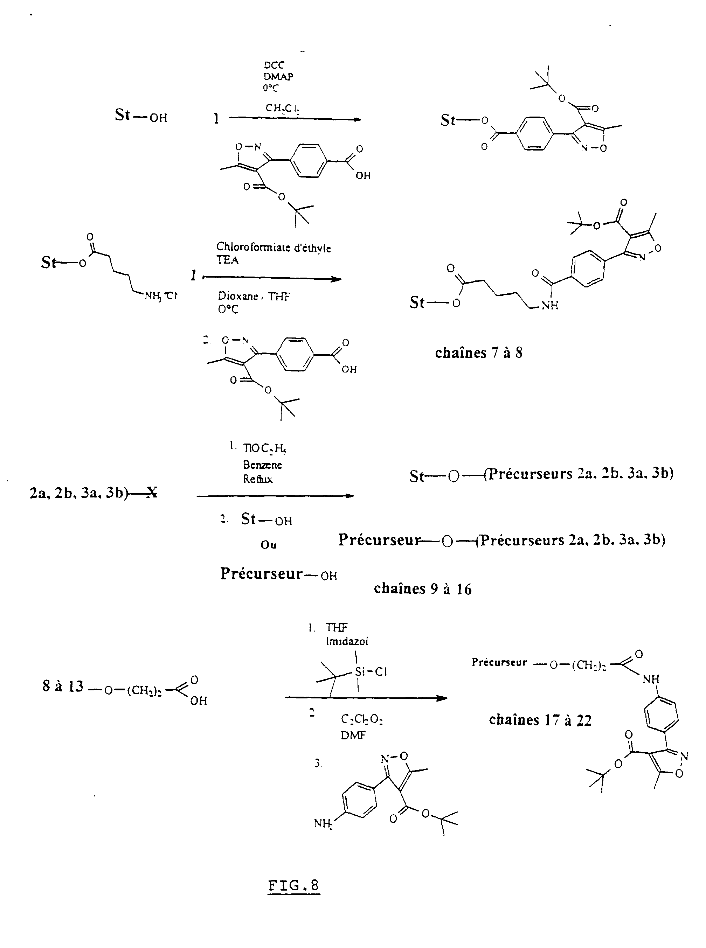

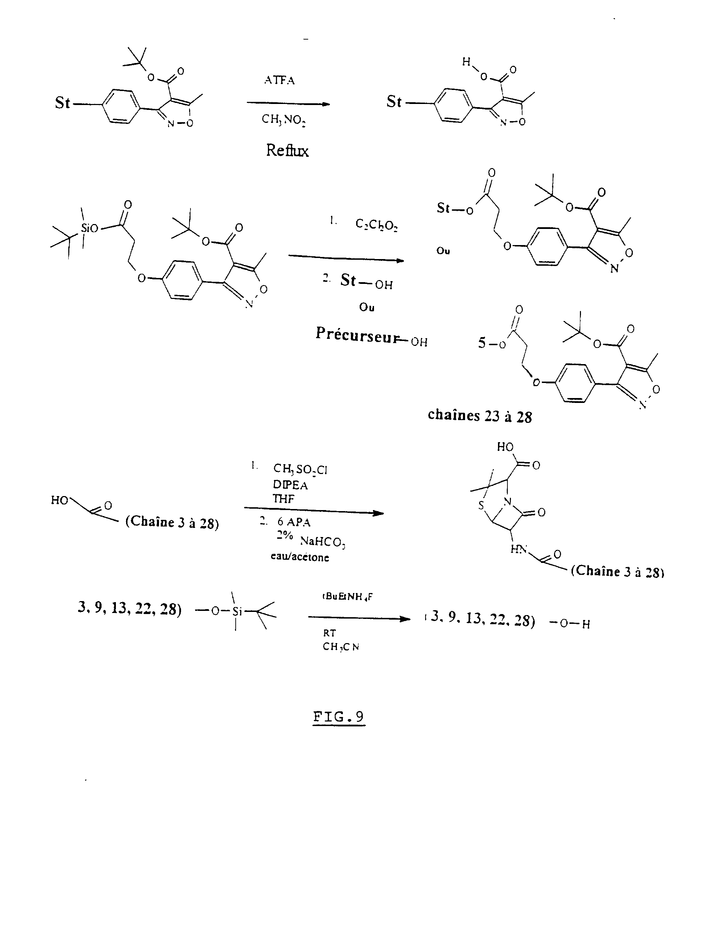

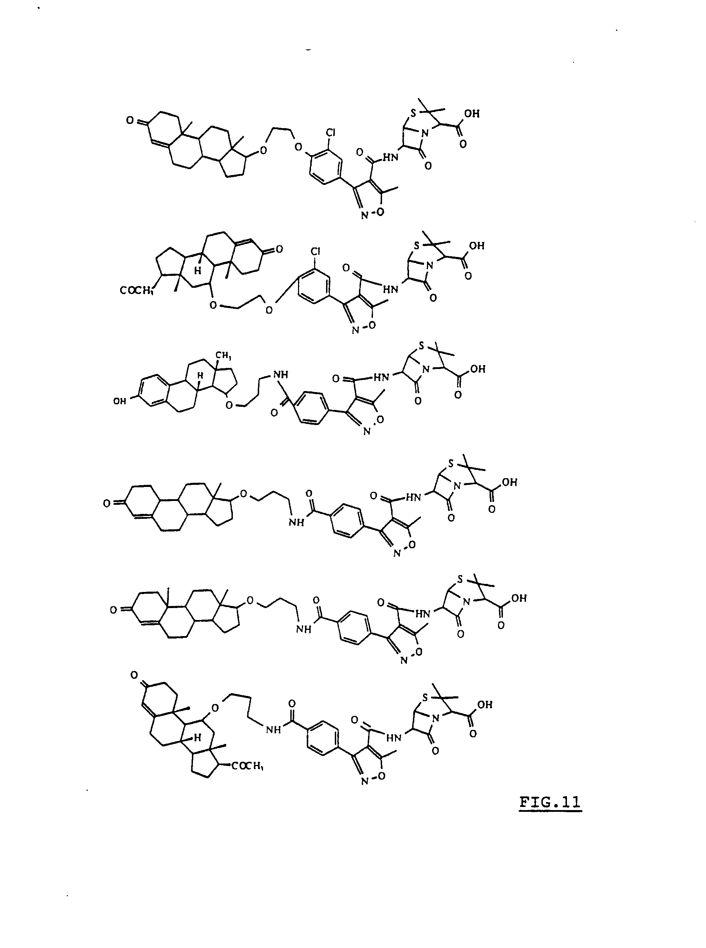

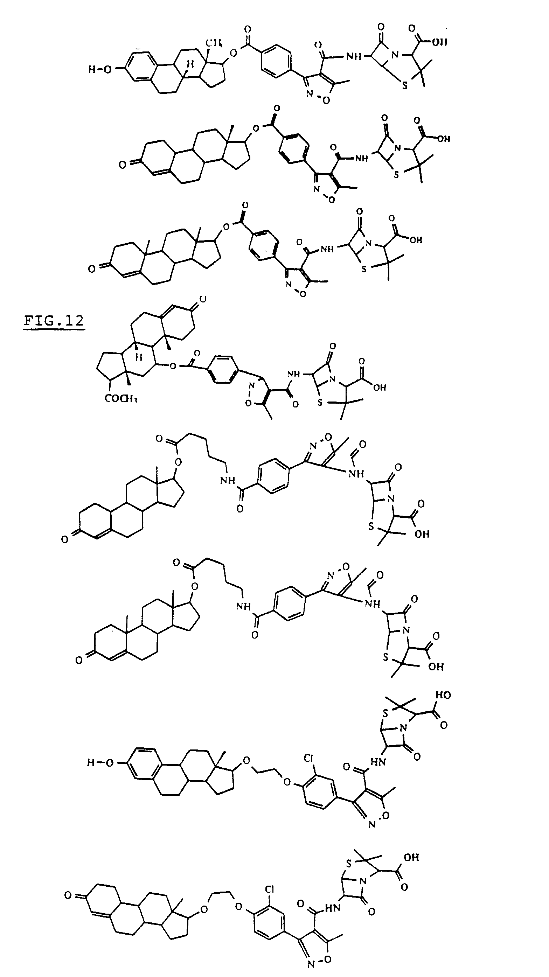

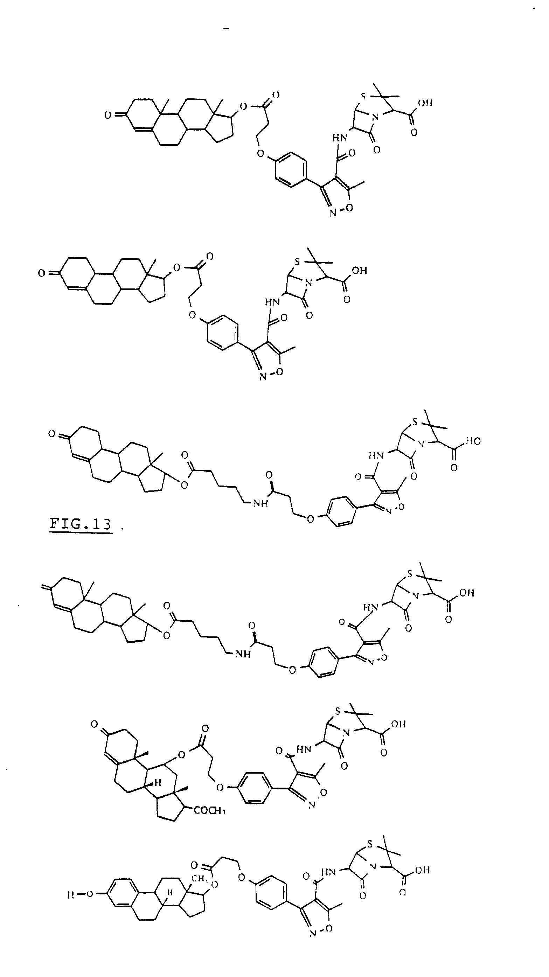

De manière à abaisser la limite de détection de la nandrolone et des autres stéroïdes, on les couple à un inhibiteur plus performant (présentant un $K_i$ particulièrement faible) et de manière à créer une gamme d'inhibiteurs aux propriétés inhibitrices variables et adaptés à différents usages requérant des pouvoirs inhibiteurs différents. La synthèse de ces produits est décrite ci-dessous. La démarche suivie pour obtenir les molécules cibles consiste à synthétiser des précurseurs comprenant la partie stéroïde et la chaîne latérale fixée à la pénicilline dûment modifiée pour permettre le couplage au stéroïde et pour générer des différences de Ki significatives. Ensuite, on forme un amide par couplage du précurseur au 6-APA pour obtenir la molécule finale. Le procédé général de synthèse exposé ci-dessous est résumé dans les figures 4 à 13.

<u>*6.1. Synthèse des précurseurs (phénylisoxazoles)*</u>

*Synthèse du 3-(4-carboxyphényl)-5-méthylisoxazole-4-carboxylate de tert-butyle (précurseur 1)*

Un équivalent de 4-carboxybenzaldéhyde (I) est mis en réaction avec deux équivalents de chlorhydrate d'hydroxylamine dans un mélange eau/méthanol ajusté à pH 4.5 avec NaOH. Après deux heures de réaction, la solution est concentrée sous vide jusqu'à précipitation du produit (II). Le précipité est filtré, lavé à l'eau glacée et redissous dans un tampon $NaHCO_3$ à pH 8. La solution de produit est purifiée au charbon, filtrée et réacidifiée par HCl. Le produit (II) qui précipite est isolé, lavé par $H_2O$ et séché sous vide.
Le produit II est dissous dans un mélange dioxanne/$CHCl_3$. La solution refroidie dans un mélange acétone/carboglace est ensuite saturée en chlore. La solution chlorée est ensuite réchauffée progressivement jusqu'à température ambiante. Après réaction complète, la solution est évaporée sous vide. Le produit obtenu (III) est dissous dans de l'éthanol et précipité par addition de pétroléine.
Un équivalent de produit III est dissous dans un mélange méthanol/acétonitrile. Après refroidissement à 0 °C, deux équivalents de tert-butyle acétoacétate de Na (IV) sont ajoutés lentement à la solution de produit III. Au terme de la réaction, la solution est additionnée d'eau acidifiée par de l'acide acétique. Le produit est extrait de la solution par du chloroforme. La phase chloroformique lavée par de l'eau est séchée sur sulfate sodique. Elle est ensuite évaporée jusqu'à concentration du produit (V).

*Synthèse des 3-[4 (2-brométhoxy)-3-chlorophényl]-5-méthylisoxazole-4-carboxylate de tert-butyle et 3-[4-(2-iodoéthoxy)-3-chlorophényl]-5-méthylisoxazole-4-carboxylate de tert-butyle (précurseurs 2a et 2b)*

La synthèse s'inspire de la préparation du produit précédent, le produit de départ étant la brométhoxybenzaldéhyde. La brométhoxybenzaldéhyde est transformée en oxyme par l'action du chlorhydrate d'hydroxylamine en milieu hydro-alcoolique dont le pH est maintenu à 5 par addition ménagée de NaOH. L'action du chlore à saturation dans le chloroforme pendant 2 heures transforme l'oxyme en chloroxyme avec substitution subséquente du noyau phényle par un atome de chlore en position ortho de l'éther. Le produit est purifié à deux reprises par chromatographie sur colonne de silice, la phase mobile étant constituée de toluène, d'acétate d'éthyle et d'acide acétique (6;1;0,1) en vue d'éliminer les composés instables qui se forment dans la réaction. Le composé stable obtenu (voir tableau 9) est ensuite mis en réaction avec 1 équivalent de sel potassique d'acétoacétate de tert-butyle. Le composé d'addition cyclise pendant la réaction pour donner le 3-[4-(2-brométhoxy)-3-chlorophényl]-5-méthylisoxazole-4-carboxylate de tert-butyle, qui peut être transformé en un dérivé iodé correspondant par réaction avec NaI en milieu acétonique.

*Synthèse des 3-[N-(3-chloropropyl) benzamide-4-yl]-5-méthylisoxazole-4-carboxylate de tert-butyle et 3-[N-(3-iodopropyl) benzamide-4-yl]-5-méthylisoxazole-4-carboxylate de tert-butyle (précurseurs 3a et 3b)*

Le 3-(4-carboxyphényl)-5-méthylisoxazole-4-carboxylate de tert-butyle est protégé par formation d'un dérivé tert-butyl diméthyl silyle sur la fonction carboxylique par réaction avec le chlorure de tert-butyl diméthyl silyle en présence d'imidazole. Le dérivé protégé est transformé en chlorure d'acide par l'action de chlorure d'oxalyle dans un mélange de diméthylformamide et de dichlorométhane. Le chlorure d'acide est ensuite formé par un amide par réaction avec la chloropropylamine introduite sous la forme de chlorhydrate dans le milieu contenant le chlorure d'acide additionné d'un excès de triéthylamine. Le dérivé chloré ainsi obtenu peut être transformé en dérivé iodé correspondant par réaction avec du NaI dans l'acétone.

*Synthèse du 3-(4-aminophényl)-5-méthylisoxazole-4-carboxylate de tert-butyle (précurseur 4)*

Le 4-nitrobenzaldéhyde est transformé en oxyme par réaction avec de l'hydroxylamine en milieu hydro-alcoolique à pH 5 (maintien du pH par addition de KOH), puis la chlorooxyme est formée par chloration à l'aide de chlore gazeux dans le chloroforme. La réaction du dérivé obtenu avec le sel potassique de l'acétoacétate de tert-butyle fournit le 3-(4-nitrophényl)-5-méthylisoxazole-4-carboxylate de tert-butyle, qui est réduit à l'aide d'hydrogène (2 bars) catalysé par le palladium sur charbon. Le dérivé aminé est isolé sous forme de chlorhydrate dans l'acétate d'éthyle.

*Synthèse du 3-[4-(2-carboxyéthoxy)phényl]-5-méthylisoxazole-4-carboxylate de tert-butyle protégé par un ester de tert-butyl diméthyl silyle (précurseur 5)*

La para hydroxybenzaldéhyde est mise en réaction avec de la propiolactone en présence de tert-butylate de potassium dans un mélange de diméthylformamide et d'acétonitrile. Le dérivé formé est converti en oxyme comme décrit ci-dessus pour les composés apparentés et protégé par réaction avec 1 équivalent de chlorure de tert-butyl diméthyl silyle pendant 2 heures dans du tétrahydrofuranne additionné d'imidazole. L'action du chlore sur le produit conduit à la chloroxyme qui est transformée en isoxazole par le sel potassique d'acétoacétate de tert-butyle selon le procédé déjà décrit.

<u>6.2. Préparation de stéroïdes en vue du couplage aux phénylisoxazoles</u>

La nandrolone et la testostérone ont été utilisées sans modification ou après addition d'un bras 5-aminovalérique ou 3-hydroxypropionique.

*Dérivés aminovalériques de nandrolone (précurseur 6) ou de testéstérone (précurseur 7)*

L'acide 5-aminovalérique est protégé par un groupe Boc par réaction de ditertbutyldicarbonate dans un mélange eau-tétrahydrofuranne en présence de NaOH. Le dérivé obtenu est mais en réaction avec un défaut de testostérone ou de nandrolone en présence d'un léger excès de dicyclohexylcarbodiimide et de diméthylaminopyridine. La réaction s'effectue dans un mélange de dioxanne et de tétrahydrofuranne à une température de O °C. La protection du groupement amine est enlevée par réaction de 4 équivalents de phénol et d'l équivalent de chlorure de triméthylsilyle dans un mélange de dichlorométhane et d'acétate d'éthyle. Le produit formé est isolé dans l'éther.

*Hémisuccinates de testostérone et de nandrolone (précurseurs 8 et 9)*

Le stéroïde est mis en réaction avec 1 équivalent d'anhydride succinique dans une solution de pyridine chauffée en autoclave à une température de 120 °C.

*6.3. Addition d'un bras propionique à la testostérone et à la nandrolone (précurseurs 10 et 11) ainsi qu'à la progestérone (précurseur 12)*

On forme le sel potassique du stéroïde (nandrolone, testostérone et 11-α-hydroxyprogestérone) par réaction d'l équivalent de stéroïde avec 1 équivalent de tert-butylate de potassium en milieu tétrahydrofuranne. Après 15 minutes, on dilue par un triple volume de diméthylformamide et on ajoute 1 équivalent de β-propiolactone.

*6.4. Addition d'un bras propionique à l'estradiol (précurseur 13)*

L'estradiol est protégé au niveau du phénol par addition d'un groupement tert-butyl diméthyl silyle : de l'estradiol et de l'éthoxyde de thallium en quantités équivalentes sont chauffés dans le benzène en éliminant en continu l'alcool qui se libère par distillation de l'azéotrope. Lorsque la réaction est complète, 1,1 équivalent de chlorure de tert-butyl diméthyl silyle est ajouté, et la réaction est poursuivie jusqu'à complétion de la substitution du phénol (précurseur 14). On forme le sel potassique du stéroïde protégé et on substitue par la chaîne propionique comme décrit ci-dessus pour les précurseurs 10 à 12.

*6.5. Formation de chaînes latérales d'oxacilline par réaction des précurseurs 1 à 5 avec les précurseurs 6 à 14*

Le précurseur 1 est activé en solution dans du dichlorométhane additionné d'une trace de diméthylformamide en présence de 5 mg % de diméthylaminopyridine et d'l équivalent soit de précurseur 14, soit de nandrolone, soit de testostérone, soit de 11-α-hydroxyprogestérone et d'un léger excès de dicyclohexylcarbodiimide ajouté progressivement dans la solution refroidie à 0 °C. Après élimination de la dicyclohexylurée, le produit est isolé dans l'éther de pétrole. La protection de l'acide isoxazole-4-carboxylique est enlevée par l'action de l'acide trifluoroacétique dans une solution de nitrométhane portée à reflux pendant 40 minutes. Le produit est purifié par chromatographie sur colonne de silice éluée par une phase mobile toluène, acétate d'éthyle et acide acétique 2;1;0,1. Les chaînes 3, 4, 5 et 6 résultent de cette préparation.

Le précurseur 1 est activé par le chloroformate d'isobutyle (1,1 équivalent) dans du dioxanne contenant une trace de diméthylformamide à une température de 0 °C en présence d'un léger excès de triéthylamine. A cette solution, on ajoute une solution dans la diméthylformamide de l'un des précurseurs 6 ou 7, et on laisse réagir en présence d'un excès de triéthylamine pour obtenir les chaînes 7 et 8 après déprotection comme ci-dessus.

L'un quelconque des précurseurs 14, nandrolone, testostérone ou 11-α-hydroxyprogestérone et de l'éthoxyde de thallium placés en quantités équivalentes dans du benzène sont chauffés en éliminant en continu l'alcool qui se libère par distillation du benzène. Lorsque la réaction est complète, 1 équivalent soit du précurseur 2a ou 2b selon la réactivité, soit du précurseur 3a ou 3b selon la réactivité est ajouté, et la réaction est poursuivie jusqu'à substitution complète. Les chaînes 9 à 16 sont obtenues par cette voie après purification sur colonne de silice avec comme phase mobile un mélange de pétroléine et de toluène de composition adaptée à la lipophilie du produit, et la déprotection est effectuée comme ci-dessus.

L'un quelconque des précurseurs 8 à 13 est transformé en ester de tert-butyl diméthyl silyle par réaction avec 1 équivalent de chlorure de tert-butyl diméthyl silyle en présence d'imidazole dans un milieu constitué de tétrahydrofuranne. Cet ester est mis en réaction dans du dichlorométhane en présence de chlorure d'oxalyle et de quantités catalytiques de diméthylformamide pour former le chlorure d'acide correspondant, qui est couplé au précurseur 4 introduit dans le milieu réactionnel en quantité équivalente. Les chaînes 17 à 22 sont produites de la sorte après déprotection selon les méthodes décrites ci-dessus. Le précurseur 5 est transformé en chlorure d'acide en milieu dichlorométhane par 1 équivalent de chlorure d'oxalyle en présence de quantités catalytiques de diméthylformamide, et mis en réaction avec l'un quelconque des précurseurs 6, 7 ou 14 ou l'un quelconque des produits suivants : nandrolone, testostérone et 11-α-hydroxyprogestérone introduits dans le milieu réactionnels en quantités équivalentes. Les chaînes 23 à 38 sont obtenues après déprotection selon la méthode déjà décrite.

*6.6. Couplage des chaînes 1 à 26 au 6-APA*

Le carboxyle en position 4 du noyau isoxazole de la chaîne est activé par le chlorure de méthane sulfonyle (1 équivalent) en milieu tétrahydrofuranne refroidi à -50 °C et contenant 1 équivalent de diisopropyléthylamine. Après réaction et retour à température ambiante, 1 équivalent d'acide 6-aminopénicillanique dissous dans une solution aqueuse de bicarbonate à 2% et dilué d'un égal volume d'acétone est introduit dans le milieu. Après élimination des solvants et purification par extraction à l'éther au départ de solutions à pH 2 et pH 8,5, on isole les conjugués 1 à 26 de la pétroléine. Toutefois, les produits 3, 9, 13, 22 et 28 doivent subir une déprotection du phénol par le mélange HF/KF à pH 5 en milieu tétrahydrofuranne.

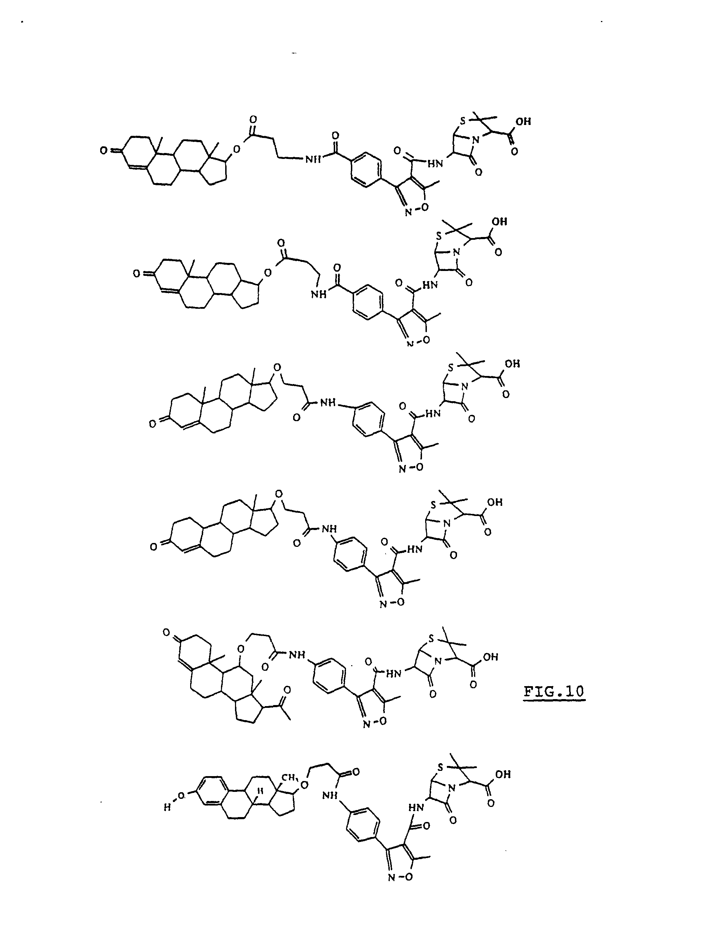

Les caractéristiques de ces conjugués (figures 10 à 13) sont reprises dans le tableau 9.

### Résultats

#### 1. Mesure d'activité enzymatique et dosage

Les mesures ont été réalisées sur des β-lactamases de souches bactériennes différentes : *Enterobacter cloacae P99, Escherichia Coli* et *Citrobacter freundii*, symbolisées par : P99, E. Coli et C. Freundii.

Les mesures sont divisées en quatre parties. La première partie comporte les mesures d'activité enzymatique en présence de quantités variables de substrat reporter (nitrocéfine) et d'enzyme (en l'absence d'inhibiteur). La seconde est une évaluation de l'activité inhibitrice des inhibiteurs (tableau 9). La troisième regroupe les mesures d'activité enzymatique pour déterminer la quantité d'anticorps à utiliser dans le dosage. La quatrième partie comprend le dosage des haptènes.

#### 2. Mesure de l'activité des enzymes

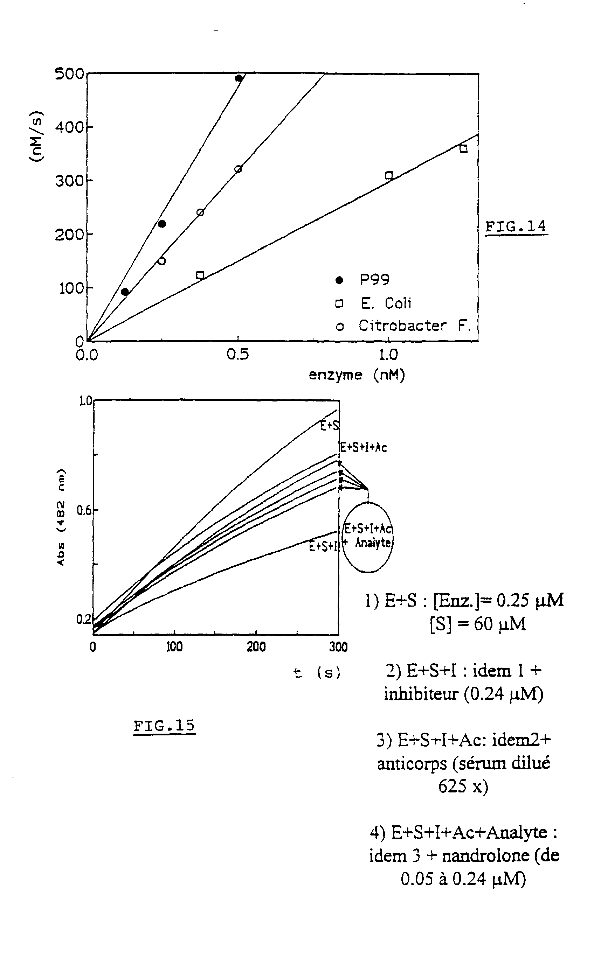

Les mesures ont été réalisées en présence de nitrocéfine. L'objectif était le calcul de la constante catalytique des enzymes, qui va servir à déterminer les quantités de substrat et d'enzyme à employer pour atteindre une vitesse enzymatique compatible avec le temps de mesure. La figure 14 montre l'évolution de la vitesse maximale des β-lactamases de P99, E. Coli et C. Freundii ($V_0$) en fonction de la concentration en enzyme ([E]) et en présence de nitrocéfine (S) à des concentrations de 60 μM pour les enzymes de P99 et E. Coli et de 150 μM pour l'enzyme de C. Freundii. La figure 14 montre que les activités enzymatiques des enzymes P99 et C. Freundii sont similaires. Les constantes catalytiques ($k_{cat}$) de ces deux enzymes, calculées sur base du graphique, sont comparables à celles de la littérature. Par contre, l'activité enzymatique de E. Coli semble relativement faible par rapport à la valeur attendue.

#### 3. Mesure de l'activité inhibitrice du carbénicillinate de nandrolone (I) (tableau 3)

A titre d'exemple, on fournit les résultats d'une étude ponctuelle effectuée sur ce composé. Les résultats présentés sur le tableau 9 donnent des valeurs obtenues selon une méthode similaire. Des mesures de la vitesse enzymatique maximale réalisées sur l'enzyme en présence de nitrocéfine et de carbénicillinate de nandrolone ($V_1$) ont servi à déterminer la constante d'inhibition ($K_i$) de l'inhibiteur. Ces mesures déterminent les concentrations en inhibiteur (I) à utiliser lors du dosage.

Tableau 3

| Enzyme | [E] (nM) | [S] (μM) | [I] (μM) | $V_1$ (nM/s) | $V_0$ (nM/s) | $V_0/V_1$ 1 | $K_i$ (nM) |
|---|---|---|---|---|---|---|---|
| **P99** | 0.0625 | 50 | 0.1 | 36 | 52 | 0.44 | 76 |
| | 0.0625 | 50 | 0.3 | 20 | 52 | 1.6 | 62 |
| | 0.0625 | 50 | 0.4 | 17 | 52 | 2.06 | 65 |
| | 0.0625 | 50 | 0.5 | 16 | 52 | 2.25 | 74 |
| | 0.25 | 60 | 0.2 | 131 | 218 | 0.66 | 88 |
| | 0.25 | 60 | 0.24 | 116 | 218 | 0.88 | 78 |
| | | | | | | | $K_i$=74 |
| **C. Freundii** | 0.375 | 60 | 0.1 | 209 | 240 | 0.15 | 80 |
| | 0.375 | 60 | 0.2 | 163 | 240 | 0.47 | 62 |
| | | | | | | | $K^i$=71 |

Tableau 3   (suite)

| Enzyme | [E] (nM) | [S] ($\mu$M) | [I] ($\mu$M) | $V_1$ (nM/s) | $V_0$ (nM/s) | $V_0/V_1$ 1 | $K_i$ (nM) |
|---|---|---|---|---|---|---|---|
| E. Coli | 1 | 150 | 0.2 | 260 | 310 | 0.19 | 800 |
| | 1 | 150 | 0.4 | 220 | 310 | 0.41 | 760 |
| | | | | | | | $K_i$=780 |

4. Mesure de l'activité inhibitrice du carbénicillinate de nandrolone en présence d'anticorps (tableau 4)

Ces mesures ont pour objectif, à titre d'exemple, l'évaluation de la restauration de la majeure partie de l'activité enzymatique en présence d'une quantité précise d'inhibiteur. Le tableau 4 donne les vitesses de réaction enzymatique ($V_{ab}$) enregistrées en présence de $\beta$-lactamases, de nitrocéfine, de carbénicillinate de nandrolone et d'anticorps anti-nandrolone. Il indique également les facteurs de dilution du sérum contenant les anticorps (dil. sérum) employés dans le milieu de mesure.

Tableau 4

| Enzyme | [S] (nM) | [E] ($\mu$M) | [I] ($\mu$M) | dil. Sérum | $V_{ab}$ (nM/s) | $V_0$ (nM/s) | $V_1$ (nM/S) |
|---|---|---|---|---|---|---|---|
| P99 | 60 | 0.25 | 0.2 | 1000 x | 170 | 218 | 131 |
| | 60 | 0.25 | 0.2 | 770 x | 189 | 218 | 131 |
| | 60 | 0.25 | 0.2 | 625 x | 200 | 218 | 131 |
| | 60 | 0.25 | 0.2 | 500 x | 200 | 218 | 131 |
| E.Coli | 150 | 1 | 0.24 | 714 x | 299 | 310 | 210 |
| | 150 | 1 | 0.24 | 625 x | 307 | 310 | 210 |
| | 150 | 1 | 1 | 500 x | 123 | 310 | 90 |
| | 150 | 1 | 1 | 200 x | 174 | 310 | 90 |
| | 150 | 1 | 1 | 140 x | 200 | 310 | 90 |
| | 150 | 1 | 1 | 125 x | 210 | 310 | 90 |
| | 150 | 1 | 1 | 100 x | 211 | 310 | 90 |

Le tableau 4 indique que pour une concentration en inhibiteur de 0.2 à 0.24 $\mu$M, une dilution du sérum d'anticorps d'un facteur de 625 semble être satisfaisante pour restaurer une vitesse proche du $V_0$. Il indique également qu'une augmentation de la concentration en inhibiteur d'un facteur 5 implique une augmentation de la concentration en sérum dans la même proportion.

5. Dosage de la nandrolone (tableau 5)

Le dosage de la nandrolone, en utilisant le conjugué 1 comme inhibiteur, a été effectué de la manière suivante : un échantillon de nandrolone est additionné d'une solution de carbénicillinate de nandrolone. Le mélange ainsi obtenu est additionné de sérum d'anticorps dilué, puis vortexé durant 30 secondes. Il est ensuite additionné d'une solution de substrat reporter, de tampon et d'enzyme. Après une brève agitation, l'absorbance du milieu à 482 nm est lue en continu durant 5 minutes.

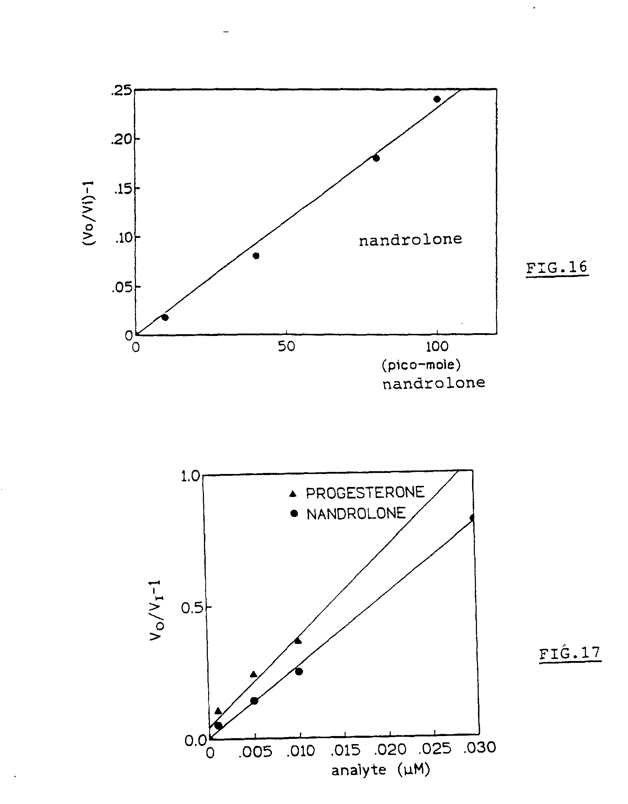

Un exemple de dosage de nandrolone est donné dans la figure 15. Il montre l'évolution de l'absorbance à 482 nm (longueur d'onde caractéristique de la nitrocéfine hydrolysée) en fonction du temps. La pente de chaque droite donne la vitesse de la réaction enzymatique. Les droites du graphique montrent l'évolution de la vitesse avec la concentration en inhibiteur libre. Les mesures ont été faites en présence de $\beta$-lactamase de P99.

Deux exemples de dosage de nandrolone exposés dans le tableau 5 ont été réalisés respectivement avec les $\beta$-lactamases de P99 et de E. coli. Ils montrent que la précision des dosages réalisés avec une même concentration en inhibiteur est supérieure avec la $\beta$-lactamase de P99. En utilisant cette dernière, la concentration minimale en nandrolone détectable dans le milieu de mesure est de l'ordre de 0.02 $\mu$M, ce qui correspond à 10 pico-moles de substance (volume de mesure = 500 $\mu$l). La concentration minimale détectable en utilisant la $\beta$-lactamase de E. coli est difficile à déterminer en raison de l'imprécision des mesures effectuées avec des concentrations inférieures à 0.05

µM (soit une quantité de 25 pico-moles).

Tableau 5

|  | [E] (µM) | [I] (µM) | dil. sérum | [Nan] (µM) | $V_{dos}$ (nM/s) | $V_{ab}$ (nM/s) | $V_i$ (nM/s) |
|---|---|---|---|---|---|---|---|
| **Dosage avec la β-lactamase de P99** | | | | | | | |
| 60 | 0.25 | 0.24 | 625 | 0.02 | 186 | 190 | 116 |
| 60 | 0.25 | 0.24 | 625 | 0.08 | 174 | 190 | 116 |
| 60 | 0.25 | 0.24 | 625 | 0.16 | 161 | 190 | 116 |
| 60 | 0.25 | 0.24 | 625 | 0.20 | 152 | 190 | 116 |
| **Dosage avec la β-lactamase de E. Coli** | | | | | | | |
| 150 | 1 | 0.24 | 625 | 0.05 | 293 | 307 | 250 |
| 150 | 1 | 0.24 | 625 | 0.10 | 283 | 307 | 250 |
| 150 | 1 | 0.24 | 625 | 0.24 | 279 | 307 | 250 |

La figure 16, décrivant l'évolution de $[(V_0/V_1)-1]$ en fonction de la quantité de nandrolone, montre une relation linéaire entre les deux paramètres. Un dosage avec la β-lactamase de E. Coli réalisé avec une concentration en inhibiteur de 1 µM et une quantité adéquate d'anticorps montre que le "range" du dosage avec cette enzyme peut être augmenté et s'accompagne d'une diminution de sensibilité. Il est donc important d'optimiser la quantité d'inhibiteur dans le but de réaliser le meilleur compromis.

6. Calcul de la quantité de substrat et d'enzyme nécessaire au dosage

Connaissant le temps de mesure du dosage, il est possible d'évaluer à partir du $k_{cat}$ les quantités d'enzyme et de substrat employées lors des dosages. Par exemple, pour les dosages présentés précédemment, un temps de mesure de 5 minutes a été fixé. Durant ces 5 minutes (dt), l'absorbance à 482 nm (maximum d'absorbance de la nitrocéfine hydrolysée) devrait idéalement grimper d'une unité (dA = 1) en l'absence d'inhibition enzymatique. Pour l'enzyme P99 ($k_{cat}$ = 780 $s^{-1}$), on désire observer un dA/dt = 1/300 ($s^{-1}$),
ce qui se traduit, compte tenu du coefficient d'extinction de la nitrocéfine hydrolysée ($\varepsilon$ = 15000 $M^{-1}$ $cm^{-1}$), par une vitesse d'hydrolyse :

$$V_0 = dA/dt = 1/(300 \times 15000) = 2.2 \ 10^{-7} \ M \ s^{-1}$$

D'un autre côté, il est admis pour des β-lactamases que :

$$V_0 = k_{cat} \ [E],$$

ce qui implique que :

$$k_{cat} \ [E] = 2.2 \ 10^{-7} \ M \ s^{-1}$$

$$[E] = 2.2 \ 10^7 \ / \ 780 = 2.8 \ 10^{-10} \ M$$

Cette concentration est proche de la concentration idéale déterminée expérimentalement (2.5 $10^{-10}$ M).
La concentration minimale en substrat pour couvrir les besoins du dosage peut également être évaluée. On sait que durant le temps de dosage (300 s) , une concentration de produit correspondant à $V_0$ dt est générée dans le milieu, soit une concentration de 300 x 2.2 $10^{-7}$ = 65 $10^{-6}$ M. Cette valeur correspond approximativement au 60 µM en substrat utilisé lors des dosages. Il faut utiliser une concentration en substrat légèrement supérieure pour assurer une vitesse maximale par saturation en substrat. D'un autre côté, il est important de limiter cette concentration pour maintenir un rapport $K_m/(K_m + [S])$ proche de l'unité.

7. Calcul de la quantité d'inhibiteur à utiliser et sensibilité du dosage

Tous les calculs théoriques concernant la quantité d'inhibiteur à utiliser et la sensibilité du dosage peuvent être réalisés à partir de l'équation :

$$\frac{V_0}{V_i} = 1 + \frac{K_m}{K_m + [S]} \frac{[I]}{K_i}$$

La quantité d'inhibiteur à utiliser dans le dosage dépend de la constante d'inhibition $K_i$. Plus cette dernière est petite, plus la quantité d'inhibiteur nécessaire pour observer une variation de vitesse enzymatique détectable est faible.

Une concentration en inhibiteur conduisant à une réduction de 50% de l'activité enzymatique semble être une bonne base pour réaliser un dosage. Le calcul dans le cas d'un dosage avec l'enzyme de P99 en présence de carbénicillinate de nandrolone ($K_i$ = 70 nM), utilisant la concentration en substrat calculée ci-dessus (65 μM), est le suivant :

$$\frac{V_0}{V_i} = 2 = 1 + \frac{25}{25 + 65} \frac{[I]}{70}$$

$$[I] = 240 \text{ nM}$$

Cette valeur correspond à la concentration utilisée lors du dosage de nandrolone avec la β-lactamase de P99.

Si on admet que l'on peut détecter aisément 10% de variation de la vitesse enzymatique, on peut détecter une quantité d'inhibiteur égale à :

$$[I] = \left(\frac{V_0}{V_i} - 1\right)\left(\frac{K_m + [S]}{K_m}\right) K_i$$

$$[I] = \left(1.1 - 1\right)\left(\frac{25 + 65}{25}\right) 70$$

$$[I] = 24 \text{ nM}$$

Dans le cas d'un dosage de nandrolone, mettant en oeuvre une quantité d'inhibiteur en présence d'une quantité stoechiométrique d'anticorps, il est possible d'évaluer la concentration de nandrolone nécessaire pour déplacer l'inhibiteur de son complexe immunologique et obtenir une concentration en inhibiteur libre de 24 nM. Dans le cas où la nandrolone a une affinité pour l'anticorps du même ordre de grandeur que l'inhibiteur, la théorie de Scatchard montre qu'une concentration de 24 nM de nandrolone déplace l'inhibiteur jusqu'à l'obtention d'une concentration en inhibiteur libre pratiquement équivalente. La limite de détection pour un volume de 500 μl est par conséquent de

$$20 \cdot 10^{-9} \cdot 500 \cdot 10^{-6} = 10 \cdot 10^{-12} \text{ mole.}$$

Cette valeur est confirmée expérimentalement par le graphique du dosage de la nandrolone représentant ($V_0/V_i$) - 1 en fonction de la quantité de nandrolone.

Les dosages réalisés montrent qu'il est possible de doser un haptène sans réaliser une étape d'incubation. La durée totale d'une mesure ne dépasse pas 7 à 8 minutes. La limite de détection de la nandrolone du système β-lactamase de P99 / carbénicillinate de nandrolone / nitrocéfine / anticorps est de 10 pico-moles.

La sensibilité est fortement améliorée par l'utilisation d'oxacillinate de nandrolone, car les valeurs de $K_i$ pour l'oxacilline (0,5 nM) sont plus avantageuses que celles de la carbénicilline (10 nM).

Des résultats de même ordre sont obtenus avec le carbénicillinate de théophylline, dont les caractéristiques cinétiques ($K_i$) sont suffisantes pour pouvoir être exploitées commercialement dans un dosage.

8. Exemples d'agents protecteurs

Le tableau 6 montre que la vitesse d'hydrolyse du substrat est influencée par la présence de sérum. En l'absence d'enzyme, on observe des vitesses d'hydrolyse du substrat (nitrocéfine) variant avec le pourcentage de sérum présent dans le milieu de mesure. Le tableau 6 montre que l'addition d'acide de sodium ($NaN_3$) ou de phénylbutazone 0.07 mg/ml réduit fortement cette vitesse. De plus, l'addition de phénylbutazone 0.07 mg/ml améliore la qualité du dosage en augmentant la disponibilité du substrat. Des exemples de dosage de la nandrolone en présence de ces deux agents protecteurs sont donnés dans ce tableau.

Tableau 6

|  | Légende |
|---|---|
| Vol. Mes. | volume final dans lequel la cinétique enzymatique est suivie |
| V | vitesse de dégradation du substrat (nitrocéfine) |
| Oenz | concentration en β-lactamase P99 dans le volume final |
| Osub | concentration en nitrocéfine dans le volume final |
| Oinh | concentration en inhibiteur dans le volume final |
| Oanal | concentration en analyte dans le volume final |
| DSA | dilution du sérum contenant les anticorps anti-nandrolone |
| %sérum | pourcentage de sérum dans le volume final |
| Tm | agent protecteur testé (PBS = tampon phosphate 0.1 M + NaCl 0.15 M) |
| Tampon | tampon utilisé lors des mesures (hépès/phos = mélange 9/1 de tampon PBS et de tampon Hépès 2.5 mM) |
| pH | pH du tampon utilisé lors des mesures |

## Tableau 6

| Vol Mes (µl) | V (µM/s) | Qenz (nM) | Qsub (nM) | Qinh (nM) | Qanal (nM) | DSA | %sérum | Traitement | Tampon | pH |
|---|---|---|---|---|---|---|---|---|---|---|
| 500 | 0.219 | 0.25 | 75 | - | - | - | 0 | - | hépès 2,5 mM | 8.2 |
| 500 | 0.143 | 0.25 | 75 | 240 | - | - | 0 | - | hépès 2,5 mM | 8.2 |
| 500 | 0.206 | 0.25 | 75 | 240 | - | 625 | 0 | - | hépès 2,5 mM | 8.2 |
| 500 | 0.14 | - | 75 | - | - | - | 24 | - | hépès 2,5 mM | 8.2 |
| 500 | 0.05 | - | 75 | - | - | - | 2 | - | hépès 2,5 mM | 8.2 |
| 500 | 0.203 | 0.25 | 75 | - | - | - | 2 | - | hépès 2,5 mM | 8.2 |
| 500 | 0.156 | 0.25 | 75 | 240 | - | - | 2 | - | hépès 2,5 mM | 8.2 |
| 500 | 0.215 | 0.25 | 75 | 240 | - | 625 | 2 | - | hépès 2,5 mM | 8.2 |
| 500 | 0.220 | 0.25 | 75 | - | - | - | 0 | - | hépès 2,5 mM | 8.2 |
| 500 | 0.110 | 0.25 | 75 | 300 | - | - | 0 | - | hépès 2,5 mM | 8.2 |
| 500 | 0.197 | 0.25 | 75 | 300 | - | 500 | 0 | - | hépès 2,5 mM | 8.2 |
| 500 | 0.196 | 0.25 | 75 | 300 | - | 454 | 0 | - | hépès 2,5 mM | 8.2 |
| 500 | 0.188 | 0.25 | 75 | 300 | - | 556 | 0 | - | hépès 2,5 mM | 8.2 |
| 500 | 0.148 | 0.25 | 75 | 300 | - | 500 | 2 | - | hépès 2,5 mM | 8.2 |
| 500 | 0.134 | 0.25 | 75 | 300 | 110 | 500 | 2 | - | hépès 2,5 mM | 8.2 |
| 500 | 0.127 | 0.25 | 75 | 300 | 400 | 500 | 2 | - | hépès 2,5 mM | 8.2 |

EP 0 897 540 B1

Tableau 6

| Vol Mes (µl) | V (µM/s) | Qenz (nM) | Qsub (nM) | Qinh (nM) | Qanal (nM) | DSA | %sérum | Traitement | Tampon | pH |
|---|---|---|---|---|---|---|---|---|---|---|
| 500 | 0.111 | 0.25 | 75 | 300 | 2000 | 500 | 2 | - | hépès 2,5 mM | 8.2 |
| 500 | 0.015 | - | 75 | - | - | - | 2 | Tp PBS + azide 0.1% | hépès/phos | 7 |
| 500 | 0.101 | 0.25 | 75 | 300 | - | - | 2 | Tp PBS + azide 0.1% | hépès/phos | 7 |
| 500 | 0.193 | 0.25 | 75 | - | - | - | 2 | Tp PBS + azide 0.1% | hépès/phos | 7 |
| 500 | 0.192 | 0.25 | 75 | 300 | - | 500 | 2 | Tp PBS + azide 0.1% | hépès/phos | 7 |
| 500 | 0.170 | 0.25 | 75 | 300 | 22 | 500 | 2 | Tp PBS + azide 0.1% | hépès/phos | 7 |
| 500 | 0.165 | 0.25 | 75 | 300 | 110 | 500 | 2 | Tp PBS + azide 0.1% | hépès/phos | 7 |
| 500 | 01.35 | 0.25 | 75 | 300 | 400 | 500 | 2 | Tp PBS + azide 0.1% | hépès/phos | 7 |
| 500 | 0.125 | 0.25 | 75 | 300 | 2000 | 500 | 2 | Tp PBS + azide 0.1% | hépès/phos | 7 |
| 450 | 0.12 | - | 75 | - | - | - | 10 | - | citrate 0.005M | 7 |
| 450 | 0.022 | - | 75 | - | - | - | 10 | phénylbutazole 0.07 mg | citrate 0.005M | 7 |
| 450 | 0.276 | 0.75 | 75 | - | - | - | 10 | phénylbutazole 0.07 mg | citrate 0.005M | 7 |
| 450 | 0.246 | 0.75 | 75 | 275 | - | - | 10 | phénylbutazole 0.07 mg | citrate 0.005M | 7 |
| 450 | 0.215 | 0.75 | 75 | 275 | 11 | 500 | 10 | phénylbutazole 0.07 mg | citrate 0.005M | 7 |
| 450 | 0.207 | 0.75 | 75 | 275 | 22 | 500 | 10 | phénylbutazole 0.07 mg | citrate 0.005M | 7 |
| 450 | 0.184 | 0.75 | 75 | 275 | 110 | 500 | 10 | phénylbutazole 0.07 mg | citrate 0.005M | 7 |

La méthode de dosage iode-amidon précitée fondée sur la génération d'iode au sein d'une solution d'empois d'amidon stabilisée pour l'iodure de cadmium trouve son application dans le dosage d'autres antibiotiques.

9. Dosage de la céphalexine (tableau 7)

On prépare 100 ml d'une solution mixte d'iodure de cadmium 0.003 N et d'iodate de potassium 0.00275 N en dissolvant respectivement 54.92 mg d'iodure de cadmium et 9.77 mg d'iodate de potassium dans une solution de tampon hépès pH 8.2 (10 mM) chargée à 1% en empois d'amidon. On prépare aussi 100 ml de solution de DTPA 0.2 M dans le tampon hépès. On prépare enfin une solution mère de céphalexine 1 mM en dissolvant 34 mg de céphalexine dans 100 ml du tampon hépès décrit ci-dessus.

*Préparation de l'échelle*

- on hydrolyse et on dilue la solution mère de céphalexine en en prélevant 1 ml que l'on traite par 4 ml de soude 0.001 M pendant 15 minutes, puis on amène à 100 ml avec le tampon hépès;
- on prépare une solution d'iode amidonnée par prélèvement de 20 ml de solution mixte, 25 ml de DTPA et 3 ml d'acide acétique glacial, puis on amène au volume par du tampon hépès (solution d'iode amidonnée);
- on réalise une échelle de concentration de 1 à 6 $\mu$M en céphalexine hydrolysée en prélevant des volumes de 100 à 600 :1 auxquels on ajoute 100 $\mu$l de la solution d'iode amidonnée. On amène au volume de 1 ml avec du tampon hépès;
- on mesure l'absorbance finale des solutions après 5 minutes à 620 nm.

Tableau 7

| Concentrations (:M) | Absorbance |
|---|---|
| 0 | 0.963 |
| 1 | 0.831 |
| 2 | 0.682 |
| 3 | 0.549 |
| 4 | 0.428 |
| 5 | 0.280 |
| 6 | 0.155 |

On obtient un coefficient de corrélation de 0.9961 et une équation de droite

$$Y = -0.1345\ X + 0.96$$

*Dosage*

- on dilue la céphalexine de façon à obtenir une concentration comprise entre 0.5 et 2 mM;
- on hydrolyse et on dilue la solution précédente selon la technique employée pour la préparation de l'échelle;
- on prélève 250 $\mu$l de la solution hydrolysée de céphalexine, on ajoute 100 $\mu$l de solution d'iode amidonnée et on amène au volume de 1 ml avec du tampon hépès;
- après 5 minutes, on lit l'absorbance à 620 nm et on calcule la concentration par rapport à l'échelle préétablie.

10. Dosage de la nandrolone en utilisant le cabénicillate de nandrolone (conjugué 1) et le mélange céphalexine-iode - amidon comme substrat reporter avec la β-lactamase de P99 (tableau 8)

Tableau 8

| S (μM) | [E] (μM) | [I] (μM) | dil. sérum | [Nan] (μM) | $V_{dos}$ (nM/s) | Vab (nM/s) | $V_i$ (nM/s) |
|---|---|---|---|---|---|---|---|
| 20 | 1 | 0.15 | 1000 | 0.0125 | 43 | 45 | 28.5 |
| 20 | 1 | 0.15 | 1000 | 0.05 | 36.3 | 45 | 28.5 |
| 20 | 1 | 0.15 | 1000 | 0.1 | 30.9 | 45 | 28.5 |
| 20 | 1 | 0.15 | 1000 | 0.125 | 28.4 | 45 | 28.5 |

La variation de coloration est observée à 620 nm qui correspond à une consommation d'iode de la céphalexine dégradée lors du processus de mesure. L'iode est généré de la manière suivante : on prélève 100 μl d'une solution mixte d'iodure de cadmium amidonné et d'iodate de potassium (CdI$_2$ 0,002 N - KIO$_3$ 0,0024 N - amidon 1,5%). On ajoute à cette solution 100 μl de solution de sel sodique de DTPA 0,1 N, et on amène le pH à 2 par addition d'HCl M. après réaction, on amène à pH 7 par addition d'une quantité suffisante de tampon Hépès 0,02 M puis on porte au volume final de 900 μl après avoir introduit les réactifs de l'immuno-dosage homogène.

| Légende tableau 9 | Colonne 2 Colonne 8 | |
|---|---|---|
| | * : FAB<br>+ : M + H<br>- : M - H | x : calculé en utilisant P99 |

Tableau 9

| Caractéristiques des conjugués de carbénicilline et d'oxacilline | | | | | | | |
|---|---|---|---|---|---|---|---|
| Conjugué | Masse | IR | | | | | $K_i$ x (nm) |
| | PP | $\nu_1$ | $\nu_2$ | $\nu_3$ | $\nu_4$ | $\nu_5$ | |
| 1 | 635*+ | 1765 | 1740 | 1725 | 1682 | 1676 | 70±43 |
| 2 | 694*+ | 1764 | 1752 | 1721 | 1673 | | 84±47 |
| 3 | 699*+ | 3294 | 1776 | 1716 | 1654 | | 24±26 |
| 4 | 699x- | 1767 | 1726 | 1717 | 1677 | 1664 | 18±23 |
| 5 | 714x- | 1788 | 1717 | 1674 | 1638 | 1279 | 22±19 |
| 6 | 759*+ | 1767 | 1744 | 1722 | 1699 | 1674 | 19±27 |
| 7 | 802*+ | 1781 | 1746 | 1719 | 1673 | | 23±18 |
| 8 | 816*+ | 1766 | 1738 | 1724 | 1680 | 1662 | 17±21 |
| 9 | 751*+ | 3302 | 1764 | 1726 | 1668 | | 14±17 |
| 10 | 753*+ | 1763 | 1725 | 1683 | 1668 | | 12±14 |
| 11 | 767*+ | 1786 | 1718 | 1675 | 1641 | | 15±16 |
| 12 | 809*+ | 1765 | 1723 | 1702 | 1668 | | 20±21 |
| 13 | 758*+ | 3299 | 1786 | 1717 | 1672 | | 18±23 |
| 14 | 760*+ | 1783 | 1720 | 1677 | | | 28±22 |
| 15 | 774*+ | 1769 | 1726 | | | | 26±27 |
| 16 | 816*+ | 1764 | 1726 | 1711 | 1670 | | 21±24 |

Tableau 9   (suite)

| Conjugué | Masse | IR | | | | | $K_i \times$ (nm) |
|---|---|---|---|---|---|---|---|
| | PP | $\nu_1$ | $\nu_2$ | $\nu_3$ | $\nu_4$ | $\nu_5$ | |
| 17 | 788*+ | 1784 | 1739 | 1720 | 1668 | | 23±19 |
| 18 | 774*+ | 1785 | 1742 | 1718 | 1671 | | 21±24 |
| 19 | 760*+ | 1787 | 1718 | 1680 | | | 29±27 |
| 20 | 746*+ | 1784 | 1717 | 1678 | | | 30±33 |
| 21 | 802*+ | 1763 | 1722 | 1703 | 1670 | | 25±23 |
| 22 | 744*+ | 3307 | 1721 | 1667 | | | 19±21 |
| 23 | 761*+ | 1768 | 1750 | 1722 | 1676 | | 21±26 |
| 24 | 747*+ | 1766 | 1742 | 1724 | 1683 | 1674 | 28±31 |
| 25 | 846*+ | 1764 | 1745 | 1724 | 1671 | | 29±26 |
| 26 | 860*+ | 1766 | 1740 | 1725 | 1669 | | 23±25 |
| 27 | 803*+ | 1765 | 1741 | 1726 | 1710 | 1674 | 26±26 |
| 28 | 745*+ | 3306 | 1765 | 1743 | 1724 | 1677 | 21±24 |

<div align="center">Caractéristiques des conjugués de carbénicilline et d'oxacilline</div>

11. Dosage de la nandrolone et de la progestérone en utilisant les oxacillinates de ces deux stéroïdes comme inhibiteur (conjugués 4 et 6)

Ce dosage est effectué comme mentionné dans l'exemple 5, et est représenté dans le tableau 10 ainsi que dans la figure 17.

Tableau 10

| [S] (µM) | [E] (µM) | [I] (µM) | dil. sérum | [Nan] (µM) | $V_{dos}$ (nM/s) | $V_{ab}$ (nM/s) | $V_i$ (nM/s) |
|---|---|---|---|---|---|---|---|
| Dosage de la nandrolone en présence d'oxacillinate de nandrolone | | | | | | | |
| 60 | 0.25 | 0.034 | 4375 | 0.001 | 190 | 200 | 110 |
| 60 | 0.25 | 0.034 | 4375 | 0.005 | 175 | 200 | 110 |
| 60 | 0.25 | 0.034 | 4375 | 0.010 | 160 | 200 | 110 |
| 60 | 0.25 | 0.034 | 4375 | 0.030 | 110 | 200 | 110 |
| Dosage de la progestérone en présence d'oxacillinate de progestérone | | | | | | | |
| 60 | 0.25 | 0.034 | 7450 | 0.001 | 185 | 205 | 105 |
| 60 | 0.25 | 0.034 | 7450 | 0.005 | 165 | 205 | 105 |
| 60 | 0.25 | 0.034 | 7450 | 0.010 | 150 | 205 | 105 |
| 60 | 0.25 | 0.034 | 7450 | 0.030 | 100 | 205 | 105 |

**Revendications**

**1.** Procédé de détection et/ou de quantification d'un haptène dans une phase homogène, dans lequel :

- on additionne une quantité connue d'un complexe inhibiteur - haptène à la solution contenant l'haptène à détecter et/ou à quantifier;
- on additionne à la solution une quantité d'anticorps correspondant à la quantité du complexe inhibiteur-haptène;
- on additionne à la solution une β-lactamase de type C possédant un site actif pour deux substrats entrant en compétition sur ledit site actif, le premier substrat étant un substrat reporter transformable en un produit détectable et/ou quantifiable, de préférence par mesure de rayonnement UV - visible, le second substrat étant le complexe inhibiteur - haptène agissant sur la vitesse d'hydrolyse du substrat reporter;

- on détecte et/ou quantifie la concentration du produit issu de la transformation du substrat reporter, la constante $K_m$ du substrat reporter étant au moins 100 fois plus élevée que la constante $K_m$ du complexe inhibiteur-haptène, et la constante $k_{cat}$ du substrat reporter étant au moins 10 fois plus élevée que la constante $k_{cat}$ du complexe inhibiteur - haptène.

**2.** Procédé selon la revendication 1, dans lequel, préalablement aux opérations précitées, on ajoute à la solution contenant l'haptène à doser des substances en vue de lever les interférences éventuelles telles que des agents protecteurs de la β-lactamase, des agents protecteurs du substrat reporter, des agents protecteurs du complexe haptène - inhibiteur ou des agents décontaminants.

**3.** Procédé selon l'une quelconque des revendications précédentes, caractérisé en ce que la constante $k_{cat}$ du substrat reporter est supérieure à $0,1\ s^{-1}$, de préférence supérieure à $10\ s^{-1}$.

**4.** Procédé selon l'une quelconque des revendications précédentes, caractérisé en ce que les constantes $K_i$ et $k_{cat}$ du complexe inhibiteur - haptène sont respectivement inférieure à $5000\ \mu M$ (de préférence inférieure à $100\ \mu M$) et inférieure à $0,1\ s^{-1}$.

**5.** Procédé selon l'une quelconque des revendications précédentes, caractérisé en ce que la β-lactamase est choisie parmi le groupe constitué par les β-lactamases issues d'*Enterobacter cloacae* Q908R et P99, de *Citrobacter freundii* et de *Escherichia coli.*

**6.** Procédé selon l'une quelconque des revendications précédentes, caractérisé en ce que l'inhibiteur et le substrat reporter sont choisis parmi le groupe constitué par les pénicillines, les céphalosporines et les antibiotiques β-lactamiques.

**7.** Procédé selon la revendication 6, caractérisé en ce que le substrat reporter est choisi parmi le groupe constitué par la céphaloridine, la nitrocéfine, la céphalothine, la céphalexine, la céphalosporine C, la céphacétrile et la céfazoline.

**8.** Procédé selon l'une quelconque des revendications précédentes, caractérisé en ce que la transformation du substrat reporter est détectée par un système de coloration amidon-iode.

**9.** Procédé selon la revendication 6, caractérisé en ce que l'inhibiteur est choisi parmi le groupe constitué par la carbénicilline, l'oxacilline, la céfuroxine, le céfotaxime et la méthicilline.

**10.** Procédé selon l'une quelconque des revendications précédentes, caractérisé en ce que l'haptène est choisi parmi le groupe constitué par les composants actifs de médicaments, les hormones, les anabolisants et/ou les drogues.

**11.** Procédé selon la revendication 10, caractérisé en ce que l'haptène est choisi parmi le groupe constitué par la testostérone, l'oestridiol, la progestérone, l'aldostérone, le cortisol, la méthylamphétamine, la méthadone, le tétrahydrocannabinol, le Δ4 androsténédione, la morphine, le DHEA sulfate, la nandrolone, la théophylline, la cocaïne et/ou leurs dérivés d'hydrolyse.

**12.** Dispositif de détection et/ou de quantification d'un haptène dans une phase homogène, caractérisé en ce qu'il comporte une β-lactamase de type C possédant un site actif pour deux substrats entrant en compétition sur ledit site actif ainsi que les deux substrats, le premier substrat étant un substrat reporter transformable en un produit détectable et/ou quantifiable, de préférence par mesure de rayonnement UV - visible, et le second substrat étant le complexe inhibiteur - haptène modulant la vitesse d'hydrolyse du complexe inhibiteur-haptène, ledit dispositif comprenant également des anticorps susceptibles de fixer ledit complexe inhibiteur-haptène, et en ce que la constante $K_m$ du substrat reporter est au moins 100 fois plus élevée grande que la constante Km du complexe inhibiteur - haptène, en ce que la constante $k_{cat}$ du substrat reporter est au moins 10 fois plus élevée que la constante $k_{cat}$ du complexe inhibiteur - haptène.

**13.** Dispositif selon la revendication 12, caractérisé en ce que la constante $k_{cat}$ du substrat reporter est supérieure à $0,1\ s^{-1}$, de préférence $10\ s^{-1}$.

**14.** Dispositif selon la revendication 12 ou 13, caractérisé en ce que les constantes $K_i$ et $k_{cat}$ du complexe inhibiteur - haptène sont respectivement inférieures à 5000 $\mu$M (de préférence 100 $\mu$M) et à 0,1 s$^{-1}$.

**15.** Dispositif selon l'une quelconque des revendications 12 à 14, caractérisé en ce que la $\beta$-lactamase est choisie parmi le groupe constitué par les $\beta$-lactamases issues d'*Enterobacter cloacae* Q908R et P99, de *Citrobacter freundii* et de *Escherichia coll.*

**16.** Dispositif selon l'une quelconque des revendications 12 à 15, caractérisé en ce que l'inhibiteur et le substrat reporter sont choisis parmi le groupe constitué par les pénicillines, les céphalosporines et les antibiotiques $\beta$-lactamiques.

**17.** Dispositif selon la revendication 16, caractérisé en ce que le substrat reporter est choisi parmi le groupe constitué par la céphaloridine, la nitrocéfine, la céphalothine, la céphalexine, la céphalosporine C, la céphacétrile et la céfazoline.

**18.** Dispositif selon l'une quelconque des revendications 12 à 17, caractérisé en ce que la transformation du substrat reporter est détectée par un système de coloration amidon-iode.

**19.** Dispositif selon l'une quelconque des revendications 16 à 18, caractérisé en ce que l'inhibiteur est choisi parmi le groupe constitué par la carbénicilline, l'oxacilline, la céfuroxine, le céfotaxime et la méthicilline.

**20.** Dispositif selon l'une quelconque des revendications 12 à 19, caractérisé en ce que l'haptène est choisi parmi le groupe constitué par les composants actifs de médicaments, les hormones, les anabolisants et/ou les drogues.

**21.** Dispositif selon la revendication 20, caractérisé en ce que l'haptène est choisi parmi le groupe constitué par la testostérone, l'oestridiol, la progestérone, l'aldostérone, le cortisol, la méthylamphétamine, la méthadone, le tétrahydrocannabinol, le $\Delta$4 androsténédione, la morphine, le DHEA sulfate, la nandrolone, la théophylline, la cocaïne et/ou leurs dérivés d'hydrolyse.

**Patentansprüche**

**1.** Verfahren zum Nachweis und/oder zur quantitativen Bestimmung eines Haptens in einer homogenen Phase, bei dem:

- eine bekannte Menge eines Inhibitor-Hapten-Komplexes zu der Lösung, die das nachzuweisende und/oder quantitativ zu bestimmende Hapten enthält, zugegeben wird;

- zu der Lösung eine Menge Antikörper zugegeben wird, die der Menge des Inhibitor-Hapten-Komplexes entspricht;

- zu der Lösung eine -Laktamase vom Typ C zugegeben wird, die eine aktive Stelle für zwei Substrate besitzt, die bei der aktiven Stelle in Wettbewerb treten, wobei das erste Substrat ein Reportersubstrat ist, das in ein vorzugsweise durch Mesung der sichtbaren UV-Strahlung nachweisbares und/oder quantitativ bestimmbares Produkt umwandelbar ist, und das zweite Substrat der Inhibitor-Hapten-Komplex ist, der auf die Hydrolysegeschwindigkeit des Reportersubstrats wirkt;

- die Konzentration des aus der Umwandlung des Reportersubstrats hervorgegangenen Produkts nachgewiesen und/oder quantitativ bestimmt wird, wobei die Konstante $K_m$ des Reportersubstrats mindestens 100-mal so groß wie die Konstante $K_m$ des Inhibitor-Hapten-Komplexes ist, und die Konstante $k_{cat}$ des Reportersubstrats mindestens 10-mal so groß wie die Konstante $k_{cat}$ des Inhibitor-Hapten-Komplexes ist.

**2.** Verfahren gemäß Anspruch 1, bei dem vor den obenerwähnten Schritten zu der Lösung, die das zu bestimmende Hapten enthält, Substanzen zugegeben werden, um die eventuellen Störungen, wie Schutzmittel der -Laktamase, Schutzmittel des Reportersubstrats, Schutzmittel des Inhibitor-Hapten-Komplexes oder dekontaminierende Mittel zu beseitigen.

**3.** Verfahren gemäß irgendeinem der vorhergehenden Ansprüche, dadurch gekennzeichnet, daß die Konstante $k_{cat}$

des Reportersubstrats größer als 0,1 s$^{-1}$, vorzugsweise größer als 10 s$^{-1}$ ist.

4. Verfahren gemäß irgendeinem der vorhergehenden Ansprüche, dadurch gekennzeichnet, daß die Konstanten K; und $k_{cat}$ des Inhibitor-Hapten-Komplexes kleiner als 5000 µM (vorzugsweise kleiner als 100 µM) bzw. kleiner als 0,1 s$^{-1}$ sind.

5. Verfahren gemäß irgendeinem der vorhergehenden Ansprüche, dadurch gekennzeichnet, daß die -Laktamase in der Gruppe gewählt wird, die von den aus Enterobacter cloacae Q908R und P99, Citrobacter freundii und Escherichia coli hervorgegangenen -Laktamasen gebildet wird.

6. Verfahren gemäß irgendeinem der vorhergehenden Ansprüche, dadurch gekennzeichnet, daß der Inhibitor und das Reportersubstrat in der Gruppe gewählt werden, die von Penicillinen, Cephalosporinen und -Laktam-Antibiotika gebildet wird.

7. Verfahren gemäß Anspruch 6, dadurch gekennzeichnet, daß das Reportersubstrat in der Gruppe gewählt wird, die von Cephaloridin, Nitrocefin, Cephalothin, Cephalexin, Cephalosporin C, Cephacetril und Cefazolin gebildet wird.

8. Verfahren gemäß irgendeinem der vorhergehenden Ansprüche, dadurch gekennzeichnet, daß die Umwandlung des Reportersubstrats durch ein Stärke-Jod-Färbesystem nachgewiesen wird.

9. Verfahren gemäß Anspruch 6, dadurch gekennzeichnet, daß der Inhibitor in der Gruppe gewählt wird, die aus Carbenicillin, Oxacillin, Cefuroxin, Cefotaxim und Methicillin gebildet wird.

10. Verfahren gemäß irgendeinem der vorhergehenden Ansprüche, dadurch gekennzeichnet, daß das Hapten in der Gruppe gewählt wird, die von den aktiven Komponenten von Medikamenten, Hormonen, Anabolika und/oder Drogen gebildet wird.

11. Verfahren gemäß Anspruch 10, dadurch gekennzeichnet, daß das Hapten in der Gruppe gewählt wird, die von Testosteron, Östridiol, Progesteron, Aldosteron, Cortisol, Methylamphetamin, Methadon, Tetrahydrocannabinol, 4-Androstenedion, Morphin, DHEA-Sulfat, Nandrolon, Theophyllin, Cocain und/oder ihren Hydrolysederivaten gebildet wird.

12. Vorrichtung zum Nachweis und/oder zur quantitativen Bestimmung eines Haptens in einer homogenen Phase, dadurch gekennzeichnet, daß sie eine -Laktamase vom Typ C, die eine aktive Stelle für zwei Substrate besitzt, die bei der aktiven Stelle in Wettbewerb treten, sowie die zwei Substrate umfaßt, wobei das erste Substrat ein Reportersubstrat ist, das in ein vorzugsweise durch Messung der sichtbaren UV-Strahlung nachweisbares und/oder quantitativ bestimmbares Produkt umwandelbar ist, und das zweite Substrat der Inhibitor-Hapten-Komplex ist, der die Hydrolysegeschwindigkeit des Inhibitor-Hapten-Komplexes moduliert, wobei die Vorrichtung außerdem Antikörper aufweist, die geeignet sind, den Inhibitor-Hapten-Komplex zu fixieren, und daß die Konstante $K_m$ des Reportersubstrats mindestens 100-mal so groß wie die Konstante $K_m$ des Inhibitor-Hapten-Komplexes ist, und daß die Konstante $k_{cat}$ des Reportersubstrats mindestens 10-mal so groß wie die Konstante $k_{cat}$ des Inhibitor-Hapten-Komplexes ist.

13. Vorrichtung gemäß Anspruch 12, dadurch gekennzeichnet, daß die Konstante $k_{cat}$ des Reportersubstrats größer als 0,1 s$^{-1}$, vorzugsweise größer als 10 s$^{-1}$ ist.

14. Vorrichtung gemäß Anspruch 12 oder 13, dadurch gekennzeichnet, daß die Konstanten K; und $k_{cat}$ des Inhibitor-Hapten-Komplexes kleiner als 5000 µM (vorzugsweise kleiner als 100 µM) bzw. kleiner als 0,1 s$^{-1}$ sind.

15. Vorrichtung gemäß irgendeinem der Ansprüche 12 bis 14, dadurch gekennzeichnet, daß die -Laktamase in der Gruppe gewählt wird, die von den aus Enterobacter cloacae Q908R und P99, Citrobacter freundii und Escherichia coli hervorgegangenen -Laktamasen gebildet wird.

16. Vorrichtung gemäß irgendeinem der Ansprüche 12 bis 15, dadurch gekennzeichnet, daß der Inhibitor und das Reportersubstrat in der Gruppe gewählt werden, die von Penicillinen, Cephalosporinen und -Laktam-Antibiotika gebildet wird.

**17.** Vorrichtung gemäß Anspruch 16, dadurch gekennzeichnet, daß das Reportersubstrat in der Gruppe gewählt wird, die von Cephaloridin, Nitrocefin, Cephalothin, Cephalexin, Cephalosporin C, Cephacetril und Cefazolin gebildet wird.

**18.** Vorrichtung gemäß irgendeinem der Ansprüche 12 bis 17, dadurch gekennzeichnet, daß die Umwandlung des Reportersubstrats durch ein Stärke-Jod-Färbesystem nachgewiesen wird.

**19.** Vorrichtung gemäß irgendeinem der Ansprüche 16 bis 18, dadurch gekennzeichnet, daß der Inhibitor in der Gruppe gewählt wird, die von Carbenicillin, Oxacillin, Cefuroxin, Cefotaxim und Methicillin gebildet wird.

**20.** Vorrichtung gemäß irgendeinem der Ansprüche 12 bis 19, dadurch gekennzeichnet, daß das Hapten in der Gruppe gewählt wird, die von den aktiven Komponenten von Medikamenten, Hormonen, Anabolika und/oder Drogen gebildet wird.

**21.** Vorrichtung gemäß Anspruch 20, dadurch gekennzeichnet, daß das Hapten in der Gruppe gewählt wird, die von Testosteron, Östridiol, Progesteron, Aldosteron, Cortisol, Methylamphetamin, Methadon, Tetrahydrocannabinol, 4-Androstenedion, Morphin, DHEA-Sulfat, Nandrolon, Theophyllin, Cocain und/oder ihren Hydrolysederivaten gebildet wird.

**Claims**

**1.** Method for detecting and/or quantifying a hapten in a homogeneous phase, in which:

- a known quantity of an inhibitor-hapten complex is added to the solution containing the hapten to be detected and/or to be quantified;
- a quantity of antibody corresponding to the quantity of the inhibitor-hapten complex is added to the solution;
- a type C $\beta$-lactamase having an active site for two substrates entering into competition on said active site is added to the solution, the first substrate being a reporter substrate capable of being transformed into a product which is detectable and/or quantifiable, preferably by visible UV radiation measurement, the second substrate being the inhibitor-hapten complex acting on the rate of hydrolysis of the reporter substrate;
- the concentration of the product resulting from the transformation of the reporter substrate is detected and/or quantified, the $K_m$ constant for the reporter substrate being at least 100 times higher than the $K_m$ constant for the inhibitor-hapten complex, and the $k_{cat}$ constant for the reporter substrate being at least 10 times higher than the $k_{cat}$ constant for the inhibitor-hapten complex.

**2.** Process according to claim 1, in which, prior to the abovementioned operations, substances are added to the solution containing the hapten to be assayed in order to remove possible interference such as agents for protecting $\beta$-lactamase, agents for protecting the reporter substrate, agents for protecting the hapten-inhibitor complex or decontaminating agents.

**3.** Process according to any one of the preceding claims, characterized in that the $k_{cat}$ constant for the reporter substrate is greater than 0.1 s$^{-1}$, preferably greater than 10 s$^{-1}$.

**4.** Process according to any one of the preceding claims, characterized in that the $K_i$ and $k_{cat}$ constants for the inhibitor-hapten complex are respectively less than 5000 $\mu$M (preferably less than 100 $\mu$M) and less than 0.1 s$^{-1}$.

**5.** Process according to any one of the preceding claims, characterized in that the $\beta$-lactamase is chosen from the group consisting of the $\beta$-lactamases obtained from *Enterobacter cloacae* Q908R and P99, from *Citrobacter freundii* and from *Escherichia coli.*

**6.** Process according to any one of the preceding claims, characterized in that the inhibitor and the reporter substrate are chosen from the group consisting of the penicillins, the cephalosporins and the $\beta$-lactam antibiotics.

**7.** Process according to claim 6, characterized in that the reporter substrate is chosen from the group consisting of cephaloridine, nitrocefin, cephalothin, cephalexin, cephalosporin C, cephacetrile and cefazolin.

**8.** Process according to any one of the preceding claims, characterized in that the transformation of the reporter substrate is detected by a starch-iodine color system.

9. Process according to claim 6, characterized in that the inhibitor is chosen from the group consisting of carbenicillin, oxacillin, cefuroxine, cefotaxime and methicillin.

10. Process according to any one of the preceding claims, characterized in that the hapten is chosen from the group consisting of medicament active components, hormones, anabolic steroids and/or drugs.

11. Process according to claim 10, characterized in that the hapten is chosen from the group consisting of testosterone, estridiol, progesterone, aldosterone, cortisol, methylamphetamine, methadone, tetrahydrocannabinol, $\Delta^4$-androstenedione, morphine, DHEA sulfate, nandrolone, theophylline, cocaine and/or their hydrolysis derivatives.

12. Device for detecting and/or quantifying a hapten in a homogeneous phase, characterized in that it comprises a type C $\beta$-lactamase having an active site for two substrates entering into competition on said active site, as well as the two substrates, the first substrate being a reporter substrate capable of being transformed into a product which is detectable and/or quantifiable, preferably by visible UV radiation measurement, and the second substrate being the inhibitor-hapten complex modulating the rate of hydrolysis of the inhibitor-hapten complex, said device also comprising antibodies capable of binding said inhibitor-hapten complex, and in that the $K_m$ constant for the reporter substrate is at least 100 times higher bigger [sic] than the $K_m$ constant for the inhibitor-hapten complex, in that the $k_{cat}$ constant for the reporter substrate is at least 10 times higher than the $k_{cat}$ constant for the inhibitor-hapten complex.

13. Device according to claim 12, characterized in that the $k_{cat}$ constant for the reporter substrate is greater than 0.1 $s^{-1}$, preferably greater than 10 $s^{-1}$.

14. Device according to claim 12 or 13, characterized in that the $K_i$ and $k_{cat}$ constants for the inhibitor-hapten complex are respectively less than 5000 $\mu$M (preferably less than 100 $\mu$M) and less than 0.1 $s^{-1}$.

15. Device according to any one of claims 12 to 14, characterized in that the $\beta$-lactamase is chosen from the group consisting of the $\beta$-lactamases obtained from *Enterobacter cloacae* Q908R and P99, from *Citrobacter freundii* and from *Escherichia coli.*

16. Device according to any one of claims 12 to 15, characterized in that the inhibitor and the reporter substrate are chosen from the group consisting of the penicillins, the cephalosporins and the $\beta$-lactam antibiotics.

17. Device according to claim 16, characterized in that the reporter substrate is chosen from the group consisting of cephaloridine, nitrocefin, cephalothin, cephalexin, cephalosporin C, cephacetrile and cefazolin.

18. Device according to any one of claims 12 to 17, characterized in that the transformation of the reporter substrate is detected by a starch-iodine color system.

19. Device according to any one of claims 16 to 18, characterized in that the inhibitor is chosen from the group consisting of carbenicillin, oxacillin, cefuroxine, cefotaxime and methicillin.

20. Device according to any one of claims 12 to 19, characterized in that the hapten is chosen from the group consisting of medicament active components, hormones, anabolic steroids and/or drugs.

21. Device according to claim 20, characterized in that the hapten is chosen from the group consisting of testosterone, estridiol, progesterone, aldosterone, cortisol, methylamphetamine, methadone, tetrahydrocannabinol, $\Delta^4$-androstenedione, morphine, DHEA sulfate, nandrolone, theophylline, cocaine and/or their hydrolysis derivatives.

$$k_{cat} \text{ grand}$$

$$K_m \gg K_I$$

**Substrat reporter**

**Produit détectable en U.V. - visible**

**Enzyme**

**ß-lactamase type C**

$$K_I \text{ petit}$$

$$k_{cat} \text{ petit}$$

$$k_{+1} \text{ élevé}$$

**Inhibiteur haptène**

**Haptène**

FIG.1

FIG.2

FIG.3

FIG.4

FIG.5

FIG.6

1. THF

2. DMF/THF

TtOC₂H₄

Benzene

FIG.7

FIG.8

FIG.9

FIG.10

FIG.11

FIG.12

FIG.13 .

FIG.14

FIG.15

1) E+S : [Enz.]= 0.25 μM
[S] = 60 μM

2) E+S+I : idem 1 +
inhibiteur (0.24 μM)

3) E+S+I+Ac: idem2+
anticorps (sérum dilué
625 x)

4) E+S+I+Ac+Analyte :
idem 3 + nandrolone (de
0.05 à 0.24 μM)

38

FIG.16

FIG.17